# NEW EUROPEAN PATENT SPECIFICATION

(11) **EP 1 861 073 B2**
(45) Date of publication and mention of the opposition decision: **18.04.2012**
(45) Mention of the grant of the patent: 06.05.2009
(21) Application number: 06710498.4
(22) Date of filing: 06.03.2006
(51) Int. Cl.: A61K 9/16, A61K 9/20, A61K 47/26, A61K 33/10, A61K 31/593

(54) **PROCESS FOR THE PRODUCTION OF CALCIUM COMPOSITIONS IN A CONTINUOUS FLUID BED**
VERFAHREN ZUR HERSTELLUNG VON CALCIUMZUSAMMENSETZUNGEN IM KONTINUIERLICHEN FLIESSBETT
PROCEDE PERMETTANT LA PRODUCTION DE COMPOSITIONS DE CALCIUM DANS UN LIT FLUIDISE CONTINU

(30) Priority: 04.03.2005 DK 200500334
(43) Date of publication of application: 05.12.2007
(73) Proprietor: Nycomed Pharma AS, 1385 Asker (NO); Nycomed Danmark ApS, 4000 Roskilde (DK)
(72) Inventor: PIENE, Jan, Yngvar, N-1387 Asker (NO); LUNDE, Kjell, Tomas, NO-1169 Oslo (NO)
(74) Representative: Johansen, Marianne
(86) International application number: PCT/IB2006/000474
(87) International publication number: WO 2006/092727

(56) References cited:
- EP-A- 0 487 774
- WO-A-00/28973
- WO-A-98/52541
- WO-A-99/65473
- WO-A-2005/115342
- FR-A- 2 717 387
- FR-A- 2 724 844
- US-A- 4 684 534
- US-A- 5 108 728
- US-A1- 2003 211 168

## Description

### Field of the invention

The present invention relates to the field of pharmaceutical formulation science, in particular with respect to methods of improving a process for production of calcium containing particulate material, wherein the calcium-containing particulate material has a content of calcium carbonate of at least 40%.

### Background

Particulate matter or a granular material may be produced by a variety of production processes in pharmaceutical manufacture including high speed mixing, dry granulation or compaction, extrusion and fluid bed processing. The most common method of granulation in pharmaceutical manufacture is by high speed mixing or high shear mixing and subsequent drying of the moist granulate in a fluid bed. This method produces a dense granulate which is appropriate for making small tablets with a high density. Fluid bed granulation is much less used as this is a more complicated process and more costly with respect to investment, process validation and running cost. The fluid bed granulation process produces a less dense granulate, which may be undesirable when ordinary tablets to be swallowed are to be manufactured. The use for calcium chewable products demands very specialized raw materials and most important a very delicate production process. The importance of combining critical characteristics of the raw materials together with a carefully selected production process has been shown for calcium chewable tablets in EP-A-1 128 815 of Nycomed Pharma AS.

This document describes a process by which the undesirably high bulk of a chewable tablet containing calcium carbonate is reduced. The reduced tablet size has been accomplished by careful selection of the physical properties of the calcium carbonate source and a fluid bed granulation and drying process. The optimal windows for the mean particle size and specific surface area were found to be 3 to 40 µm and 0.1 to 1.2 m²/g respectively for the preferred qualities of calcium carbonate. The choice of particle size range was especially important in order to achieve a satisfactory chewability and dispersion in the mouth where as the specific surface area was important in order to accomplish an efficient or short processing time during the granulation and drying phase in a fluid bed. The fluid bed granulation step has resulted in a very homogenous distribution of the binder, which in turn results in a rapid dispersion of the table when chewed but also very good consolidation properties during the tableting step. This last property is very important for the productivity of high speed tableting machines to ensure maximum output and a minimum demand for cleaning and maintenance of tablet tooling.

However, the use of fluid bed granulation and drying raise some problems that remain unsolved. These problems are related to the design of the batch fluid bed equipment itself but also to the control of the batch fluid bed process and the execution of a batch recipe.

The experienced batch related fluid bed problems are laid down in the below section:
- Regular problems are the adherence of a powder or granulate to inner parts of fluid bed apparatus, to the spray nozzles and air filters. Another problem has been fine powder particles being lodged beneath the product screen in the lower plenum where the inlet air passes into the fluid bed. In addition to the gradual deposition of powder layers in the expansion chamber this causes a need for regular cleaning.
- During the course of a batch recipe of calcium granulate there have been problems in ensuring a satisfactory fluidization during the end of the granulation step and the beginning of the drying step. Especially during the summer season
where the dehumidifying capacity is at its limits there have been problems with insufficient drying and lump formation in the product container. This causes a significant problem of granulate batches, which are not according to specification with respect to the moisture content which is too high. In order to compensate for this it has been necessary to adjust the concentration of the binder in the granulation liquid and to increase the air capacity which in turn causes extra wear and tear on the exhaust filters.
Thus, an unsatisfactory reproducibility with respect to the moisture content and particle size/distribution of the granulate is experienced in a batch process even during constant ambient conditions with respect to inlet air humidity and absolute moisture. There is thus a need to increase the robustness of the process especially in the case of variations of the inlet air humidity.
- The in-process sampling procedure for a batch fluid bed is a problem due to the fact that the calcium granulates as it comes out of the product container may not be homogenous with respect to the moisture content and particle size distribution. This is especially the case when there has been a problem with too humid conditions in the fluid bed with a resultant insufficient drying and lump formation.
- The batch process has been found to be very sensitive with respect to the specific surface area, particle size and shape of the calcium-containing compound to be agglomerated. An increase in the specific surface area or a different particle shape of calcium carbonate will often require reformulation work and a new set of qualification and validation batches to be produced.
- Further, the process is a batch process where the raw material must be loaded and removed after each batch, slowing down the production rate considerably.

Continuous fluid bed granulation and drying is mainly a process that has been utilized for high volume or mono-product processes in chemical and food industry. The pharmaceutical industry has not utilized this technology to any great extent due to fact that pharmaceutical production normally requires rapid batch and recipe changes, a rigorous cleaning between product changes and regulatory difficulties in the definition of batch size.

### Summary of the invention

It has surprisingly been found, that use of a continuous fluid bed apparatus solves most of the problems with adherence of the granulate to inner parts of fluid bed apparatus, uncontrolled lump formation at high relative humidities in the product container, unsatisfactory reproducibility with respect to moisture content and particle size/distribution and problems related to inhomogeneous samples during in process sampling. This also reduces the time-consuming loading/unloading process of the apparatus and in particular minimizes the need for cleaning.

It has also surprisingly been found that the mean particle size can be effectively varied over a wide particle size range in the continuous fluid bed process by carefully controlling the moisture load, which the powder mixture is exposed to.

Furthermore it has surprisingly been found that the continuous fluid bed process is much less sensitive to processing difficulties and variation in moisture content and particle size/distribution of the granulate when different sources of calcium are employed with different physical characteristics like specific surface area, particle size/distribution and particle shape. Especially, it has been found that it is possible to obtain a much narrower particle size distribution by using a process involving continuous fluid bed than by a process using batch fluid bed. Such a narrow particle size distribution is of particular advantage in order to obtain homogeneous powder mixtures.

Thus, the present invention relates to a process for producing a particulate material comprising a calcium-containing compound, the process comprises granulating afluidized composition comprising the calcium-containing compound optionally together with one or more pharmaceutically acceptable excipients under fluidizing conditions in a continuous fluid bed apparatus.

The present method has been found to be an efficient and cost-effective method that, furthermore, has the advantages that a particulate material is prepared that has controllable moisture content that has controllable particle size and particle size distribution. Moreover, the method is a robust process, which means that once the process parameters for the fluid bed process are found and the fluid bed process is started, no or only minor adjustments are required.

The process of the invention comprises the steps of
i) continuously feeding the composition to a zone of the continuous fluid bed apparatus with a feed rate (kg/h),
ii) continuously transferring the fluidized composition throughout one or more zones of the continuous fluid bed apparatus with a rate corresponding to that of the feed rate,
iii) continuously wetting the composition by spraying a granulation liquid to the fluidized composition with a spray load (kg solventlh),
iv) continuously drying the wetted composition, and
v) continuously collecting the thus obtained particulate material with an output rate corresponding to that of the feed rate.

The particulate material normally has a content of calcium compound of at least about 40 % by weight, normally at least about 60% w/w such as at least about 70% w/w, at least about 80% w/w, at least about 90% w/w or at least about 95% w/w.

Furthermore, the method may comprise a step of compressing the particulate material obtained optionally together with one or more pharmaceutically acceptable excipients and/or one or more therapeutically, prophylactically and/or diagnostically active substance to form tablets.

In another aspect, the process of the invention provides a particulate material comprising a calcium-containing compound and one or more pharmaceutically acceptable excipients, wherein the SPAN value is at the most about 2.3 such as, e.g., at the most about 2.25, at the most about 2.1, at the most about 2 or at the most about 1.9. As seen from the examples herein, a span value in the range of from about 1.7 to about 1.9 is obtainable by the process according to the invention, while the span value obtained relating to the preparation of a particulate material having the same composition but using a batch fluid bed process results in a span value of about 2.6-2.7. Accordingly, shift from a batch fluid bed to a continuous fluid bed decreases the span value about 30%. The SPAN value is calculated as [D(v, 0.9) - D(v, 0.1)]/D(v, 0.5), The particle size analysis is performed on a Malvern Mastersizer S long bench apparatus where D(v, 0.1), D(v, 0.5) and D(v, 0.9) give the particle sizes for which 10%, 50% and 90% of the particles by volume have sizes below the given values. D(v, 0.5) is the mean particle size.
As explained herein, a continuous fluid bed process according to the invention results in particulate material that has a very narrow particle size distribution as evidenced by the span value.

In a further aspect, the invention relates to the use of a particulate material as defined herein or obtained by a process as defined herein for the preparation of a dosage form.

Also described is a process for producing a solid dosage form comprising a calcium-containing compound, said process comprises steps of
i) optionally mixing a particulate material obtained as defined herein with one or more pharmaceutically acceptable excipients to produce a powder mixture that has a content of the calcium-containing compound of at least 60 % by weight; and
ii) processing the particulate material or the powder mixture into the solid dosage form.

### Detailed description of the invention

As mentioned above, there is a need for improving the process of batch fluid bed technology.

A batch fluid bed is based on the principle that four distinct unit-process phases take place in one and the same compartment, namely preheating/mixing, granulation, drying and cooling. Thus, in the same processing compartment the set-points for the critical process parameters will have to be changed frequently in order to carry out a batch recipe. All in all the batch process requires a thorough control and monitoring of the process to ensure that the critical process parameters are within the validated process windows at all times. This is due to the fact that the batch process requires frequent stepwise adjustments of the critical process parameters as the batch recipe is carried out. In other words, the process parameters employed during the preheating/mixing step are different from those employed in the granulation step that again are different from those employed in the drying step and in the cooling step. Although changes in process parameters normally are carried out automatically, even small changes may be detrimental to the success of the process.

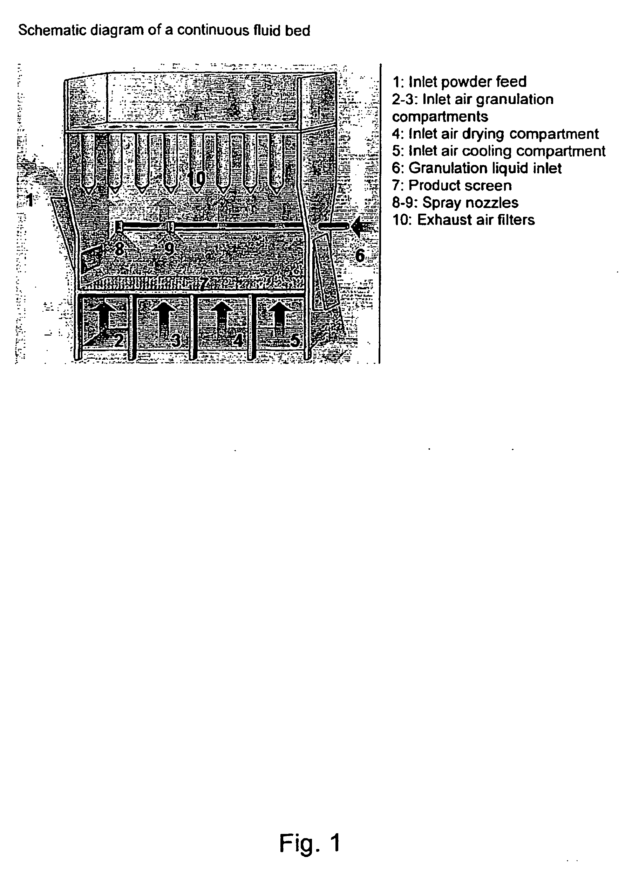

In the continuous fluid bed process each process step takes place with its own inlet air compartment or zones, which may be a more proper term, as the individual zones may not be strictly separated from each other. This is visualized in figure 1 showing a continuous fluid bed apparatus having four inlet air compartments, in this case two granulation zones and one drying and one cooling zone. The inlet air of each zone can be individually controlled with respect to the air volume, absolute moisture content and temperature, which ensures that these critical process parameters are not subject to any changes during the whole process - the one and same zone has the same function, i.e. carries out a particular unit-process during the whole process and accordingly, there is no need for adjusting any process parameter to another unit-process. Accordingly, all critical process parameters remain unchanged during the continuous process.

The continuous fluid bed process is a steady state process, which means that at any point in the horizontal fluidized bed there will be stationary conditions. This gives a much better process control than a batch process, as it is not necessary to adjust the critical process parameters in each compartment. This results in fewer fluctuations of the critical process parameters and a better process control.

Furthermore, a continuous fluid bed has both a much lower bed height and a lower amount of product per m² of product screen compared to a batch process. This allows for more fluidization air per kg product and gives more flexibility with respect to adjusting the moisture load and drying conditions. The result is a more controlled fluidization with much less chance of uncontrolled agglomeration and uneven wetting of the powder bed.

The present inventors have found that use of continuous fluid bed apparatus solves most of the problems with adherence of the granulate to the inner parts of fluid bed apparatus, uncontrolled lump formation at high relative humidity in the product container and inhomogeneous sampling of particulate material. The use of a continuous fluid bed apparatus also reduces the time-consuming loading/unloading process and in particular minimizes the need for cleaning.

Another advantage of using continuous fluid bed apparatus is that the mean particle size can be effectively varied over a wide particle size range in the continuous fluid bed process by carefully controlling the moisture load that the powder mixture is exposed to.

The in-process sampling is well controlled in a continuous process as a sample is taken out of the continuous and homogenous product stream on the outlet side.

The continuous fluid bed process thus offers a better process control and a more reproducible process with fewer variations in product characteristics like bulk density, particle size/distribution and moisture content when compared to a batch process.

Thus, the present invention relates to a process for producing a particulate material comprising a calcium-containing compound, the process comprises granulating a fluidized composition comprising the calcium-containing compound optionally together with one or more pharmaceutically acceptable excipients under fluidizing conditions in a continuous fluid bed apparatus.

In Figure 1 is shown a schematic drawing of a continuous fluid bed apparatus. As seen from the figure, the composition is fed into the apparatus and the individual unit-processes takes place in zones within the continuous fluid bed. Accordingly, a process according to the invention comprises the steps of
i) continuously feeding the composition to a zone of the continuous fluid bed apparatus with a feed rate (kg/h),
ii) continuously transferring the fluidized composition throughout one or more zones of the continuous fluid bed apparatus with a rate corresponding to that of the feed rate,
iii) continuously wetting the composition by spraying a granulation liquid to the fluidized composition with a spray load (kg solvent/h),
iv) continuously drying the wetted composition, and
v) continuously colleting the thus obtained particulate material with an output rate corresponding to that of the feed rate.

In some cases cooling may also be applied to the dried composition before it is collected.

In general, the steps are performed in two or more zones of the continuous fluid bed apparatus, although this may differ from apparatus to apparatus. In those cases, where two or more zones are used, steps i) and iv) and/or steps iii) and iv) are performed in different zones of the continuous fluid bed apparatus.

Thus, viewed from one aspect, the present invention discloses a process as described above, whereby adherence of the processed material to inner parts of the continuous fluid bed apparatus is substantially avoided.

In still another embodiment the present invention discloses a process as described above whereby the particulate material obtained is a free-flowing, non-adherent particulate material.

In one embodiment of the present invention the low moisture content corresponds to a range of from about 0.1 % to about 0.5% w/w such as, e.g., from about 0.1 % to about 0.3%.

The following is a description of the critical process parameters for a continuous fluid bed process and in particular the typical processing windows for the critical process parameters for the granulation of a calcium-containing compound in a range of continuous fluid bed models with different capacities or outputs. The feed rate of the calcium-containing composition applied to the process will depend on the particular apparatus used as laid down in the table below. The specific values stated below are based on the preparation of a specific particulate material as described in the examples herein. In general, depending on the particular apparatus employed, the composition of the particular particulate material to be prepared and the desired mean particle size e.g. the feed rate, the spray load, the air flow and the bed load may be varied within certain limits such as e.g. ± 50%, ±40%, ±30%, ±20% or ±10%.

| Production model | Product screen area (m²) | Feed rate (kg/hr) | Bed load (kg) | Spray load (g H₂O/min) | Air flow (m³/hr) | Linear air velocity (m/s) | Feed rate/spray load |
|---|---|---|---|---|---|---|---|
| Heinen WT 4/13 | 0.52 | 75 (50-100) | 75 | 159 | 1000 | 0.53 | 7.85 |
| Heinen WT 5/58 | 2.9 | 500 (250-500) | 500 | 1060 | 5000 | 0.54 | 7.85 |
| Heinen WT 10/58 | 5.8 | 500-1000 | 1000 | 2120 | 10800 | 0.52 | 7.85 |

The production scale equipment as laid down in the above table is from Heinen and it must be understood that the same processing conditions and relationships will apply to other types of continuous fluid bed granulation and drying equipment from suppliers like Glatt and Niro/Aeromatic. The table gives the relationship between the critical process parameters for a particular product in a continuous fluid bed with the following definitions:
Product screen area (m²): It is the specific fluid bed area (m²/kg/h), which is important when scaling up or down in a continuous fluid bed. The value should be constant for each individual processing section (agglomeration, drying and cooling).
Feed rate (kg/hr): This is proportional to the output and denotes the production capacity for a given production scale equipment.
Bed load (kg): This denotes the actual amount of material inside the equipment at any moment.
Spray load (g H₂O/min): This is the amount of pure water (or solvent) sprayed on to the moving bed when corrected for the dry material in the added binder.
Airflow (m³/hr): This is the total volume of air going through the sum of the process compartments in the processing equipment.
Linear air velocity (m/s): This is air speed, which the fluidised powder bed experience at the bottom of the product container near the bottom screen.
Feed rate (kg/h)/spray load (kg/h): This is an index, which is constant for a particular product composition and independent of the production scale equipment used. The spray load is chosen such as to give a granulate with optimal granule characteristics with respect to particle size/distribution, bulk density and moisture content.
Retention time (hr): This is defined as bed load over feed rate.

Moreover, the following definitions are used herein:
The term "continuous fluid bed process" is intended to mean a process, wherein each unit-process phase takes place with its own inlet air compartment. This is visualised in figure 1 by one or two granulation, one drying and one cooling compartment. The inlet air of each compartment can be individually controlled with respect to the absolute moisture content and temperature, which ensures that these critical process parameters can stay unchanged during the continuous process.

The term "particulate material" is intended to be synonymous with granulate material or simply granulate.

The term "formulated" is intended to relate to the selection of excipients, carriers, vehicles, solvents, co-solvents, preservatives, colouring agents, flavouring agents and so forth in the preparation of a medicament using said composition.
In the present context, the term "pharmaceutically acceptable excipient" is intended to denote any material, which is inert in the sense that it substantially does not have any therapeutic and/or prophylactic effect *per se*. A pharmaceutically acceptable excipient may be added to the active drug substance with the purpose of making it possible to obtain a pharmaceutical formulation, which has acceptable technical properties.

The following set-point processing parameters are important to adjust properly e.g. when changing composition of the particulate material to be prepared or in connection with e.g. up- or down-scaling between different continuous fluid bed equipment sizes:
i) air velocity,
ii) inlet air temperature,
iii) inlet air humidity,
iv) bed height,
v) feed rate (kg/h)/spray load (kg solvent/h),
vi) atomizing pressure for the nozzle(s) employed,
vii) number of nozzles/product screen area.

The present inventors have found that at least one of these parameters must be kept constant during up- or down-scaling (in this context the term "up- and down-scaling" is used to denote a shift in apparatus size and not just an increase or decrease in the bed load of a particular apparatus). In general, the most important parameter to keep constant is the ratio between the feed rate (kg/h) and the spray load (kg solvent/h). In further embodiment of the invention two or more such as, e.g., 3 or more, 4 or more, 5 or more, 6 or more or all s of the set-point processing parameters are kept constant during up- or down-scaling.

When an optimal set of critical process parameters has been found for one production size then up- or down-scaling is straight forward due to the fact that the above process parameters are kept constant.

Besides the up- and down-scaling possibility, the process according to the invention is relatively robust with respect to set-point processing parameters towards changes in the mean particle size of the particular calcium-containing compound employed. This means that in the case that calcium carbonate is employed as calcium-containing compound then it is possible to select different qualities such as, e.g. qualities having different mean particle sizes without any significant change in the set-point processing parameters, if any. The same applies for qualities having different bulk densities.

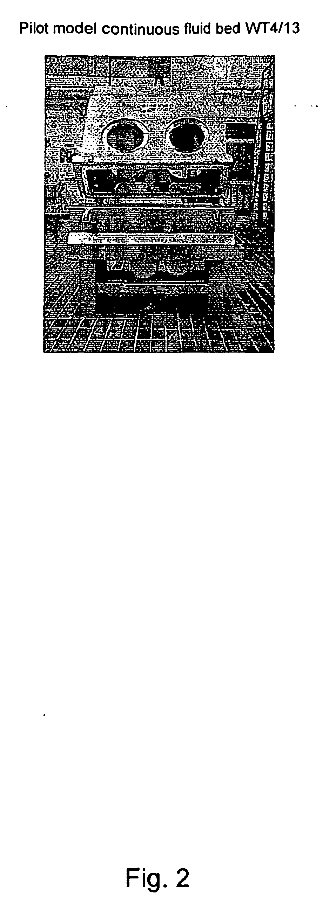

In a specific embodiment a series of trials have been carried out on a Heinen WT 4/13 pilot model continuous fluid bed. WT4/13 continuous fluid bed apparatus consists of a sieve bottom plate of about 0.52 m² and with three inlet air sections that can be controlled separately with respect to air volume, temperature and humidity. The equipment has a capacity in the range of up to 100 kg agglomerated product per hour, an air throughput of maximum 1800 m³/h and a water evaporation rate of maximum 70 kg/h. The three spray nozzles were positioned after each other above the fluidised bed (top spray) in the centre of the bed where the first nozzle sprayed at an angle against the direction of the moving bed and the two next nozzles at an angle with the moving bed. Two nozzles were positioned in the first compartment whereas the third nozzle was positioned in the second compartment. Figure 2 shows a photograph of WT4/13.

The trials showed an index of feed rate/spray load in a range of 4.5 to 45, such as, e.g. from about 5 to about 40, from about 5 to about 35, from about 5 to about 30, from about 5 to about 25, from about 6 to about 20, from about 6 to about 15, from about 6 to about 10 or from about 7 to about 8. Preferably, from 6.8 to 22.5 and most preferably about 7.9.

### Granulation step

The ingredients fed to the first zone are normally in the form of a composition comprising one or more calcium-containing compounds. The composition may be exclusively composed of one or more calcium-containing compounds, in particular of one calcium-containing compound, or it may be composed of a mixture of the calcium-containing compound(s), one or more pharmaceutically acceptable excipients and, if relevant, one or more therapeutically, prophylactically or diagnostically active substances such as, e.g., those mentioned herein. The pharmaceutically acceptable excipients are materials normally employed such as, e.g. fillers, diluents etc. Specific examples can be found under the heading "Pharmaceutically acceptable excipients" and in the examples herein.

In a specific aspect, the particulate material obtained by a method according to the invention comprises
i) one or more calcium-containing compounds,
ii) one or more binders
iii) optionally, one or more pharmaceutically acceptable excipients
iv) optionally, one or more sweetening agents.

Normally, the one or more pharmaceutically acceptable excipients and/or sweetening agents, if present, are contained in the composition containing the calcium-containing compound that is granulated in the continuous fluid bed. As discussed above, the binder may also be present in this composition.

More specifically, the particulate material comprises
i) from about 40% to about 99.9% w/w of one or more calcium containing compounds,
ii) from about 0.1 % to about 30% w/w of one or more binders
iii) from about 0.1 to about 15% w/w of one or more pharmaceutically acceptable excipients, if present, and
iv) from about 5% to about 50% w/w of one or more sweetening agents, if present, provided that the total concentration does not exceed 100%.

The feed rate depends on the size of the product screen area of the continuous fluid bed apparatus. The feed rate is normally in a range from 25 to 200 kg/h, such as e.g. 50 to 100 kg/h, in particular 60 to 80 kg/h and preferably about 75 kg/h for an apparatus with a bed load of about 75 kg. The retention time is one hour with a resultant bed load of 75 kg. As it appears from the table above, the feed rate may be much higher, e.g. from about 500 to about 1000 kg/h when larger equipment sizes are employed.

As mentioned before, the ratio between the feed rate (kg/h) of the composition comprising the calcium-containing compound and the spray load (kg solvent/h) of the granulation liquid is important in order to obtain the desired product. In specific embodiments the ratio is in a range of from about 4 to about 45 such as, e.g., in a range of from about 6 to about 23, from about 6 to about 12, from about 6 to about 10, from about 6.5 to about 8.5 or from about 7 to about 8.

Granulation takes place in the first two zones with position of the nozzles as described above The number of nozzles may vary, cf. above. In a specific embodiment, three nozzles are used and they are positioned above the pulsating powder bed to deliver a fine atomised spray with the granulation liquid (which in a specific embodiment contains the dissolved binder) with a resulting agglomeration of particles to form larger granules or agglomerates.

### Granulation liquid

The granulation of the fluidized composition is performed by means of a granulation liquid that is applied to the fluidized composition comprising the calcium-containing compound.

In order to build up agglomerates of the powder mixture that is fed to the continuous fluid bed apparatus, it is generally required to use a binder. In one aspect - as exemplified in the examples herein - the granulation liquid comprises a pharmaceutically acceptable binder. However, suitable agglomeration may also be obtained by application of the granulation liquid to a fluidized composition that comprises a pharmaceutically acceptable binder. The latter case may be of specific interest when the composition comprises e.g. a sugar alcohol that has binding properties. Within the scope of the invention is also the use of a binder in the granulation liquid as well as in the fluidized composition.

The granulation liquid may also contain one or more further pharmaceutically acceptable excipients or additive such as, e.g., soluble or intense sweeteners.

The granulation liquid is normally based on water although organic solvents like e.g. alcohol (e.g. ethanol, propanol or isopropanol) may be added.

In a specific embodiment the binder is selected from water-soluble binders.

Examples of suitable binders are dextrins, maltodextrins (e.g. Lode® 5 and Lodex® 10), dextrose, fructose, glucose, inositol, erythritol, isomalt, lactitol, lactose (e.g., spray-dried lactose, α-lactose, β-lactose, Tabletose®, various grades of Pharmatose®, Microtose or Fast-Floc®), maltitol, maltose, mannitol, sorbitol, sucrose, tagatose, trehalose, xylitol, low-substituted hydroxypropylcellulose (e.g LH 11, LH 20, LH 21, LH 22, LH 30, LH 31, LH 32 available from Shin-Etsu Chemical Co.), microcrystalline cellulose (e.g., various grades of Avicel®, such as Avicel® PH101, Avicel® PH102 or Avicel® PH105, Elcema® P100, Emcocel®, Vivacel®, Ming Tai® and Solka-Floc®), starches or modified starches (e.g. potato starch, maize starch, rice starch, pre-gelatinised starch), polyvinylpyrrolidone, polyvinylpyrrolidone/vinyl acetate copolymer, agar (e.g. sodium alginate), carboxyalkylcellulose, dextrates, gelatine, gummi arabicum, hydroxypropyl cellulose, hydroxypropylmethylcellulose, methylcellulose, polyethylene glycol, polyethylene oxide, polysaccharides e.g. dextran, soy polysaccharide.

Preferably, the granulation liquid is an aqueous medium. In the case where the binder is included in the granulation liquid, the granulation liquid is prepared by dissolving or dispersing the binder in water. Alternatively the binder can be admixed in a dry form to the powder.

The present inventors have found that the spray rate or more correctly the spray load of the granulation liquid has a major impact on the mean particle size, whereas the inlet temperature and binder concentration in the granulation liquid have minor effect on particle size. The subsequent drying and, if needed, cooling steps have little influence on the mean particle size.

Accordingly, in a specific embodiment the invention provides a method for controlling the mean particle size of the particulate material obtained by a process according to the present invention by proper adjustment of the spray load and/or the moisture content of inlet air. In general, the particle size increases with increasing spray load (if an aqueous medium is used in the granulation liquid) or with increasing moisture content of the inlet air (see e.g. the examples herein).

Normally, a particulate material obtained by a process according to the invention has a mean particle size that is suitable for use within the pharmaceutical field especially in connection with further processing of the particulate material into a solid dosage form. To be more specific, the mean particle size of the particulate material obtained is normally in a range of from about 100 to about 500 µm such as, e.g., from about 100 to about 400 µm, from about 100 to about 350 µm or from about 100 to about 300 µm.

In one embodiment the present invention relates to a process, wherein a very narrow size distribution of the particulate material is obtained. A narrow size distribution is important in order to secure an acceptable homogeneity when the particulate material is mixed with other solid pharmaceutically acceptable excipient e.g. for the manufacture of solid dosage forms. A suitable homogeneity ensures that the correct dose is contained in each dosage form, thus, enabling fulfilling of the official requirements with respect to e.g. dose variation. Moreover, a mean particle size which coincides with the mean particle size and particle size distribution of vitamin D₃ has been found to be important in order to ensure a satisfactory homogeneity of vitamin D₃ in the particulate material or the tableting end-mix. A narrow distribution for the particle size is characterised by a low value for the span value as defined below.

The SPAN value is calculated as [D(v, 0.9) - D(v, 0.1)]/D(v, 0.5), The particle size analysis is performed on a Malvern Mastersizer S long bench apparatus where D(v, 0.1), D(v, 0.5) and D(v, 0.9) give the particle sizes for which 10%, 50% and 90% of the particles by volume have sizes below the given values. D(v, 0.5) is the mean particle size.

In one embodiment of the present invention, the span value is at the most about 2.3 such as, e.g., at the most about 2.25, at the most about 2.1, at the most about 2 or at the most 1.9.

Furthermore, a narrow particle size distribution can be obtained irrespective of the kind and size of the continuous fluid bed apparatus employed, and/or the particular calcium carbonate employed.

Accordingly, the particulate material obtained normally has a SPAN value of at the most about 2.3 such as, e.g., at the most about 2.25, at the most about 2.1 or at the most about 2 irrespective of the bed size of the continuous fluid bed apparatus employed, provided that the composition of the particular particulate material is the same and the ratio between the feed rate (kg/h) and the spray load (kg/h) is kept substantially constant, and/or the particulate material obtained has a SPAN value of at the most about 2.3 such as, e.g., at the most about 2.25, at the most about 2.1 or at the most about 2 irrespective of the particle size of the particular calcium-containing compound employed provided that all other conditions including the set-points for processing parameters are substantially identical, and/or the particulate material obtained has a SPAN value of at the most about 2.3 such as, e.g., at the most about 2.25, at the most about 2.1 or at the most about 2 irrespective of the bulk density of the particular calcium-containing compound employed provided all other conditions including the set-points for processing parameters are substantially identical.

As mentioned above and as exemplified in the examples, the granulated composition obtained under by a process according to the invention has a SPAN value that is smaller than that obtained when granulating the same composition with the same granulation liquid, but in a batch fluid bed apparatus. In general, the SPAN value obtained is about 10% or more such as, e.g., about 15% or more, about 20% or more or about 30% or more smaller than that obtained using a batch fluid bed apparatus.

In order to obtain an efficient and fast granulation (i.e. agglomeration) of the calcium-containing composition the present inventors have found that a critical parameter is the moisture load which the powder mixture is exposed to from the spray nozzles where the granulation liquid optionally containing an acceptable binder. In the examples herein, a process according to the invention is illustrated by the preparation of a particulate material containing a calcium-containing compound, wherein the calcium-containing compound in admixture with one or more pharmaceutically acceptable excipients is granulated with an aqueous solution of polyvinylpyrrolidone as an example of a binder. In such situations, where an aqueous solution of the binder is employed, the concentration of the pharmaceutically acceptable binder in the dispersion, preferably a solution that is sprayed onto the powder mixture is at the most about 50% w/w such as at the most about 33% w/w.

### Drying and cooling step

Drying takes place normally in another zone than that used for application of the granulation liquid. During drying the moisture inside the granules is evaporated by the aid of diffusion. It is favourable to practice a high inlet temperature in order to ensure a quick drying process with a resultant low moisture content below 0.5% in the outlet granular material. The drying inlet air is in the range of 45 to 100°C and more preferably 70 to 100°C.

In a pilot model continuous fluid bed like the WT 4/13 there is not a separate cooling compartment. However in a production model there will be a fourth section dedicated to cooling and where the temperature of the granular material is taken down to a product temperature between 40 and 50°C.

The most favourable set points for the critical process variables as demonstrated in examples 4 and 5 were as follows:
- Inlet air volume: 1000 m³/h (at approx. 35°C)
- Absolute moisture content of inlet air: 4 g/kg
- Concentration of PVP in granulation liquid: 15%
- Feed rate of powder mixture: 75 kg/h
- Retention time 1 hour
- Spray rate with three nozzles: 187. 5 g/min
- Inlet air temperature in all three inlet air compartments: 80°C

### Other aspects of the invention

The particulate material obtained by the proves of the invention comprises
i) one or more calcium containing compounds,
ii) one or more binders
iii) optionally, one or more pharmaceutically acceptable excipients
iv) optionally, one or more sweetening agents.

More specifically, the particulate material comprises
i) from about 40% to about 99.9% w/w of one or more calcium containing compounds,
ii) from about 0.1 % to about 30% w/w of one or more binders,
iii) from about 0.1 to about 15% w/w of one or more pharmaceutically acceptable excipients, if present, and
iv) from about 5% to about 50% w/w of one or more sweetening agents, if present, provided that the total concentration does not exceed 100%.

Moreover, the invention relates to the use of a particulate material as obtained by a process as defined herein for the preparation of a dosage form. Of specific interest is the use of a particulate material together with a vitamin D containing composition for the preparation of a dosage form.

In a further aspect, the invention described a process for producing a solid dosage form comprising a calcium-containing compound, said process comprises steps of
i) optionally mixing a particulate material obtained as defined herein with one or more pharmaceutically acceptable excipients to produce a powder mixture that has a content of the calcium-containing compound of at least 60 % by weight; and
ii) processing the particulate material or the powder mixture into the solid dosage form.

More specifically, the present invention described a process for producing a pharmaceutically acceptable dosage form, said process comprises steps of
i) optionally mixing the particulate material obtained by employing of the continuous fluid bed process according to the present invention with one or more further components, i.e. one or more further active substances and/or one or more pharmaceutically acceptable excipients to produce a second particulate material, preferably having a content-of calcium compound of at least 60 % by weight; and
ii) optionally compressing said first or second particulate material to form tablets.

In one embodiment of the present invention said further component mixed with the particulate material obtained from the continuous fluid bed process is a therapeutically, prophylactically and/or diagnostically active agent.

In a preferred embodiment of the present invention said further component mixed with the particulate material obtained from the continuous fluid bed process is Vitamin D.

In a particular embodiment of the present invention said further component mixed with the particulate material obtained from the continuous fluid bed process is Vitamin D₃ or Vitamin D₂ or derivatives thereof, or mixtures thereof.

In one embodiment the present invention discloses a process as described above, wherein the homogeneity of the further component such as Vitamin D₃ is very high in both the particulate material used to compress tablets (i.e. in the admixture of the particulate material obtained from the continuous fluid bed process and the one or more further components) and the resulting tablets. The resulting particulate material (i.e. the material used for the compression) contains a much smaller coarse fraction compared to granulates from a batch process and is thus better suited with respect to the blending efficiency and prevention of subsequent segregation.

A suitable solid dosage form is tablets, capsules or sachets including chewable, suckable and swallowable tablets.

In a specific embodiment, the solid dosage form is in the form of tablets that optionally are provided with a coating.

All details and particulars mentioned under the main aspect of the invention (process for preparing a particulate material) apply *mutatis mutandis* to the other aspects of the invention.

### Calcium-containing compound

The calcium-containing compound used in a process according to the invention is a physiologically tolerable calcium-containing compound that is therapeutically and/or prophylactically active.

Calcium is essential for a number of key functions in the body, both as ionized calcium and a calcium complex (Campell AK.Clin Sci 1987; 72:1-10). Cell behaviour and growth are regulated by calcium. In association with troponin, calcium controls muscle contraction and relaxation (Ebashi S. Proc R Soc Lond 1980; 207:259-86).

Calcium selective channels are a universal feature of the cell membrane and the electrical activity of nerve tissue and the discharge of neurosecretory granules are a function of the balance between intracellular and extra cellular calcium levels (Burgoyne RD. Biochim Biophys Acta 1984;779:201-16). The secretion of hormones and the activity of key enzymes and proteins are dependent on calcium. Finally calcium as a calcium phosphate complex confers rigidity and strength on the skeleton (Boskey AL. Springer, 1988:171-26). Because bone contains over 99% of the total body calcium, skeletal calcium also serves as the major long-term calcium reservoir.

Calcium salts such as, e.g., calcium carbonate is used as a source of calcium especially for patients suffering from or at risk of osteoporosis. Moreover, calcium carbonate is used as an acid-neutralizing agent in antacid tablets.

As mentioned above, calcium has a number of important functions within the mammalian body in particular in humans. Furthermore, in many animal models, chronic low calcium intake produces osteopenia. The osteopenia affects cancellous bone more than cortical bone and may not be completely reversible with calcium supplementation. If the animal is growing reduced calcium intake leads to stunting. In the premature human neonate the higher the calcium intake, the greater the increase in skeletal calcium accretion which, if high enough, can equal gestational calcium retention. During growth chronic calcium deficiency causes rickets. Calcium supplements in both pre- and postpubertal healthy children leads to increased bone mass. In adolescents the higher the calcium intake, the greater the calcium retention, with the highest retention occurring just after menarche. Taken together, these data suggest that in children and adolescents considered to be taking an adequate intake of calcium, peak bone mass can be optimized by supplementing the diet with calcium. The mechanisms involved in optimizing deposition of calcium in the skeleton during growth are unknown. They are probably innate properties of the mineralization process that ensures optimal calcification of the osteoid if calcium supplies are high. The factors responsible for stunting of growth in states of calcium deficiency are also unknown but clearly involve growth factors regulating skeletal size.

In adults calcium supplementation reduces the rate of age-related bone loss (Dawson-Hughes B. Am J Clin Nut 1991;54:S274-80). Calcium supplements are important for individuals who cannot or will nor achieve optimal calcium intakes from food. Furthermore, calcium supplement is important in the prevention and treatment of osteoporosis etc.

Furthermore, calcium may have anticancer actions within the colon. Several preliminary studies have shown high calcium diets or intake of calcium supplementation is associated with reduced colon rectal cancer. There is increasing evidence that calcium in combination with acetylsalicylic acid (ASA) and other non-steroidal anti-inflammatory drugs (NSAIDS) reduce the risk the risk of colorectal cancer.

Recent research studies suggest that calcium might relieve premenstrual syndrome (PMS). Some researchers believe that disruptions in calcium regulation are an underlying factor in the development of PMS symptoms. In one study, half the women of a 466 person group of pre-menopausal women from across the U.S. were tracked for three menstrual cycles and were given 1200 mg of calcium supplements daily throughout the cycle. The final results showed that 48% of the women who took placebo had PMS related symptoms. Only 30% of those receiving calcium tablets did.

Calcium salts like e.g. calcium carbonate is used in tablets and due to the high dose of calcium required, such tablets are often in the form of chewable tablets. It is a challenge to formulate e.g. chewable tablets containing a calcium salt, which tablets have a pleasant taste and an acceptable mouth feel without the characteristic dominating taste or feeling of chalk.

The calcium carbonate may be used alone or in combination with other calcium-containing compounds such as e.g. bisglycino calcium, calcium acetate, calcium chloride, calcium citrate, calcium citrate malate, calcium cornate, calcium fluoride, calcium glubionate, calcium gluconate, calcium glycerophosphate, calcium hydrogen phosphate, calcium hydroxyapatite, calcium lactate, calcium lactobionate, calcium lactogluconate, calcium phosphate, calcium pidolate, calcium stearate and tricalcium phosphate. Other calcium sources may be water-soluble calcium salts, or complexes like e.g. calcium alginate, calcium-EDTA and the like or organic compounds containing calcium like e.g. calcium organophosphates. Use of bone meal, dolomite and other unrefined calcium sources is discouraged because these sources may contain lead and other toxic contaminants. However, such sources may be relevant if they are purified to a desired degree.

Normally, a dosage form made according to the invention contains an amount of the calcium-containing compound corresponding to from about 100 to about 1000 mg Ca such as, e.g., from about 150 to about 800 mg, from about 200 to about 700 mg, from about 200 to about 600 mg or from about 200 to about 500 mg Ca.

### Calcium carbonate

Calcium carbonate can be in three different crystal structures: calcite, aragonite and vaterite. Mineralogically, these are specific mineral phases, which relate to the distinct arrangement of the calcium, carbon and oxygen atoms in the crystal structure. These distinct phases influence the shape and symmetry of the crystal forms. For example, calcite is available in four different shapes: scalenohedral, prismatic, spherical and rhombohedral, and aragonit crystals can be obtained as e.g. discrete or clustered needle-like shapes. Other shapes are also available such as, e.g., cubic shapes (Scoralite 1A + B from Scora).

A suitable quality of calcium carbonate is calcium carbonate having a mean particle size of 60 µm or less such as, e.g., 50 µm or less or 40 µm or less.

Furthermore, an interesting quality of calcium carbonate has a bulk density below 2 g/mL.

Calcium carbonate 2064 Merck (available from Merck, Darmstadt, Germany) that has a mean particle size of 10 - 30 µm, an apparent bulk density of 0.4 to 0.7 g/mL, and a specific surface area of 0.3 m2/g;

Calcium carbonate 2069 Merck (available from Merck, Darmstadt, Germany) that has a mean particle size of approx. 3.9 µm, and an apparent bulk density of 0.4 to 0.7 g/mL;

Scoralite 1A (available from Scora Watrigant SA, France) has a mean particle size of 5 to 20 µm, an apparent bulk density of 0.7 to 1.0 g/mL, and a specific surface area of 0.6 m2/g;

Scoralite 1 B (available from Scora Watrigant SA, France) has a mean particle size of 10 -25 µm, an apparent bulk density of 0.9 to 1.2 g/mL, and a specific surface area of 0.4 to 0.6 m2/g;

Scoralite 1A + B (available from Scora Watrigant SA, France) have a mean particle size of 7 - 25 µm, an apparent bulk density of 0.7 to 1.2 g/mL, and a specific surface area of 0.35 to 0.8 m2/g;

Pharmacarb LL (available from Chr. Hansen, Mahawah New Jersey) L has a mean particle size of 12 - 16 µm, an apparent bulk density of 1.0 to 1.5 g/mL, and a specific surface area of 0.7 m2/g;

Sturcal L, Sturcal H, Sturcal F and Sturcal M (available from Specialty Minerals, Bethlehem, Pensylvania); Sturcal L has a mean particle size of approx. 7 µm, an apparent bulk density of 0.78 to 0.96 g/mL, Sturcal L consists of scalenohedral shaped crystals;

Sturcal H has a mean particle size of approx. 4 µm, an apparent bulk density of 0.48 to 0.61 g/mL;

Sturcal F has a mean particle size of approx. 2.5 µm, an apparent bulk density of 0.32 to 0.43 g/mL;

Sturcal M has a mean particle size of 7 µm, an apparent bulk density of 0.7 to 1.0 g/mL, and a specific surface area of 1.0 m2/g;

Mikhart 10, SPL, 15, 40 and 65 (available from Provencale, Provencale, France); Mikhart 10 has a mean particle size of 10 µm,
Mikhart SPL has a mean particle size of 20 µm,
Mikhart 15 has a mean particle size of 17 µm,
Mikhart 40 has a mean particle size of 30 µm, an apparent bulk density of 1.1 to 1.5 g/mL;
Mikhart 65 has a mean particle size of 60 µm, an apparent bulk density of 1.25 to 1.7 g/mL;

Omyapure 35, (available from Omya S.A.S, Paris, France) has a mean particle size of 5 - 30 µm, and a specific surface area of 2.9 m²/g;

Socal P2PHV (available from Solvay, Brussels, Belgium) has a mean particle size of 1.5 µm, an apparent bulk density of 0.28 g/mL, and a specific surface area of 7.0 m²/g;

Calci Pure 250 Heavy, Calci Pure 250 Extra Heavy and Calci Pure GCC HD 212 with a mean particle size of 10-30 µm, an apparent bulk density of 0.9 - 1.2 µ/ml, and a specific surface area of 0.7 m²/g (available from Particle Dynamic Inc., St. Louis Montana).

The content of the calcium-containing compound in a tablet made according to the present invention is in a range from about 40% to about 100% w/w such as, e.g., from about 45% to about 98% w/w, from about 50% to about 95% w/w, from about 55% to about 90% w/w or at least about 60% w/w, at least about 65% w/w, at least about 70% w/w or at least about 75% w/w.

Normally, the dose of calcium for therapeutic or prophylactic purposes is from about 350 mg (e.g. newborn) to about 1200 mg (lactating women) daily. The amount of the calcium-containing compound in the tablets can be adjusted to that the tablets are suitable for administration 1-4 times daily, preferably once or twice daily.

In a specific embodiment the calcium-containing compound used in a process of the invention is calcium carbonate such as calcium carbonate mentioned above.

### D vitamin or other active substances

A granulate or tablet made according to the invention may comprise a further therapeutically and/or prophylactically active substance, or it may contain one or more nutrients such as, e.g. one or more vitamins or minerals. Of specific interest are e.g. vitamin B, vitamin C, vitamin D and/or vitamin K and minerals like e.g. zink, magnesium, selenium etc.

Of particular interest are one or more D-vitamin compounds such as, e.g., Vitamin D₂ (ergocalciferol) and Vitamin D₃ (cholecalciferol) including dry vitamin D₃, 100 CWS available from Roche and dry vitamin D₃ 100 GFP available from BASF.

In addition to its action on calcium and skeletal homeostasis, vitamin D is involved in the regulation of several major systems in the body. The actions of vitamin D are medicated at the genome by a complex formed by 1,25-(OH)₂ vitamin D mainly produced in the kidney, with the vitamin D receptor (VDR). The latter is widely distributed in many cell types. The 1,25-(OH)₂ vitamin D/VDR complex has important regulatory roles in cell differentiation and in the immune system. Some of these actions are probably dependant on the ability of certain tissues other than the kidney to produce 1,25-(OH)₂ vitamin D locally and act as a paracrine (Adams JS et al. Endocrinology 1996;137:4514-7).

In humans, deficiency of vitamin D results in rickets in children and osteomalacia in adults. The basic abnormality is a delay in the rate of mineralization off osteoid as it is laid down by the osteoblast (Peacock M. London Livingstone, 1993:83-118). It is not clear whether this delay is due to a failure of a 1,25-(OH)₂ vitamin D-dependant mechanism in the osteoblast or to reduced supplies of calcium and phosphate secondary to malabsorption or a combination of both. Accompanying the mineralization delay, there is reduced supply of calcium and phosphate, severe secondary hyperparathyroidism with hypocalcaemia and hypophosphatemia and increased bone turnover.

Vitamin D insufficiency, the preclinical phase of vitamin D deficiency, also causes a reduced calcium supply and secondary hyperparathyroidism, albeit of a milder degree than found with deficiency. If this state remains chronic, osteopenia results. The biochemical process underlying this state of calcium insufficiency is probably inappropriate level of 1,25-(OH)₂ vitamin D due to a reduction in its substrate 25-OHD (Francis RM et al. Eur J Clin Invest 1983; 13:391-6). The state of vitamin D insufficiency is most commonly found in the elderly. With age there is a decrease in serum 25-OH vitamin D due to decreased sunlight exposure and possible to decreased skin synthesis. Furthermore, in the elderly the condition is exacerbated by a decrease in calcium intake and a paradoxical decrease in calcium absorption. The reduction in renal function with age giving rise to reduced renal 1,25-(OH)₂ vitamin D production may be a contributing factor. There are a number of studies of the effects of vitamin D supplementation on bone loss in the elderly. Some are without calcium supplementation and others are with calcium supplementation. It appears from the studies that although vitamin D supplementation is necessary to reverse deficiency and insufficiency, it is even more important as far as the skeleton is concerned to provide calcium supplementation since the major skeletal defect is calcium deficiency. In literature based on clinical trials, recent findings suggest trends of need for higher doses of vitamin D for the elderly patients (Compston JE. BMJ 1998;317:1466-67). An open quasi-randomised study of annual injections of 150.000-300.000 IU of vitamin D (corresponding to approx. 400-800 IU/day) showed a significant reduction in overall fracture rate but not in the rate of hip fracture in treated patients (Heikinheimo RJ et al. Calcif Tissue Int 1992; 51:105-110).

As it appears from above, a combination of calcium and vitamin D is of interest. The recommended Daily Allowance (RDA) of calcium and vitamin D₃ are as follows (European Commission. Report on osteoporosis in the European Community. Action for prevention. Office for official Publications of the European Communities, Luxembourg 1998):

| Group Age | (years) | Calcium (mg)* | Vitamin D₃ (µg) |
|---|---|---|---|
| Newborn | 0-0.5 | 400 | 10-25 |
| | 0.5-1.0 | 360-400 | 10-25 |
| | | | |
| Children | 1.0-3.0 | 400-600 | 10 |
| | 4.0-7.0 | 450-600 | 0-10 |
| | 8.0-10 | 550-700 | 0-10 |
| | | | |
| Men | 11-17 | 900-1000 | 0-10 |
| | 18-24 | 900-1000 | 0-15 |
| | 25-65 | 700-800 | 0-10 |
| | 65+ | 700-800 | 10 |
| | | | |
| Women | 11-17 | 900-1000 | 0-15 |
| | 18-24 | 900-1000 | 0-10 |
| | 25-50 | 700-800 | 0-10 |
| | 51-65 | 800 | 0-10 |
| | 65+ | 700-800 | 10 |
| | | | |
| Pregnant | | 700-900 | 10 |
| | | | |
| Lactating | | 1200 | 10 |

| | | | |
|---|---|---|---|
| * RDA of calcium varies from country to country and is being re-evaluated in many countries. | | | |

Vitamin D is very sensitive towards humidity and is subject to degradation. Therefore, vitamin D is often administered in a protective matrix. Accordingly, when tablets are prepared containing a vitamin D it is of utmost importance that the compression forces applied during the tabletting step do not decrease the protective effect of the matrix and thereby impair the stability of vitamin D. To this end, the combination of the various ingredients in a granulate or tablet made according to the invention has proved to be very suitable in those cases where vitamin D also is incorporated into the composition as it is possible to employ a relatively low compression force during tabletting and still achieve a tablet with suitable mechanical strength (crushing strength, friability etc.).

### Other active ingredients

Examples include isoflavones, vitamin K, vitamin C, vitamin B6 and oligosaccharides such as inulin and oligofructose. Isoflavones exhibit a weak oestrogenic effect and can thus increase bone density in post-menopausal women. Isoflavones are available under the trade name Novasoy 400 from ADM Nutraceutical, Illinois, USA. Novasoy 400 contains 40% isoflavones and will typically be used in an amount sufficient to provide 25 to 100 mg isoflavone/dosage. Isoflavones may be included in the second granulate; however as Novasoy 400 is a relatively cohesive powder it is preferred that it be included in the first granulate in order to ensure that it is uniformly distributed. Vitamin K (more especially vitamin K₁) may improve biochemical markers of bone formation and bone density and low concentrations of vitamin K, have been associated with low bone mineral density and bone fractures. Vitamin K, is available from Roche as Dry Vitamin K, 5% SD, a dry substance containing 5% vitamin K₁. Typically vitamin K, will be used in a quantity sufficient to provide 0.05 to 5 mg vitamin K₁/dosage. Vitamin C and vitamin B6 (available from Roche, Takeda and BASF amongst others) function as co-factors in the formation of collagen, the main component of the organic matrix of bone. Vitamin C and vitamin B6 will typically be used in quantities sufficient to provide 60 to 200 mg vitamin C/dosage and 1.6 to 4.8 mg vitamin B6/dosage respectively.

Oligosaccharides have been shown to facilitate and increase calcium absorption and may typically be used in quantities sufficient to provide 0.3 to 5 g oligosaccharide/dosage. In general it is desirable that a total of at least 5g oligosaccharide is administered daily to facilitate calcium uptake and to obtain a pre-biotic effect.

Where an active component is used which forms a minor part of the overall granulate, e.g. vitamin D, it is general preferred to produce a premix of such a component and the first granulate before mixing the premix and the remaining required quantity of the first granulate. This ensures uniform distribution of the minor component in the second granulate.
In a specific embodiment, the invention can be used to provide a tablet comprising
i) a calcium-containing compound as an active substance,
ii) a vitamin D, and
iii) optionally one or more pharmaceutically acceptable excipients.

More specifically, the tablet may comprise
i) at least 200 mg of the calcium-containing compound (normal range 200-1500 mg),
ii) at least 5 µg of vitamin D (normal range 5-100 µg 1 µg = 40 IU), and
iii) optionally one or more pharmaceutically acceptable excipients.

In a specific embodiment, the invention can be used to provide a tablet comprising
i) from about 50% to about 90% w/w of the calcium-containing compound,
ii) from about 0.00029%o about 0.0122 w/w of a vitamin D, and
iii) optionally one or more pharmaceutically acceptable excipients
with the proviso that the total amount of ingredients corresponds to about 100% w/w.

In particular, the tablet may comprise
i) from about 50% to about 90% w/w of the calcium-containing compound,
ii) from about 5 to about 30% w/w of a sweetening agent,
iii) from about 0.12% to about 4.9 % w/w of a vitamin D including a protective matrix, as delivered by supplier.
iv) optionally one or more pharmaceutically acceptable excipients
with the proviso that the total amount of ingredients corresponds to about 100% w/w.

### Preparation of a tablet

The process according to the invention may also comprise compression of a particulate material obtained as described herein optionally in admixture with one or more pharmaceutically acceptable excipients.

In general, tablets can be prepared by any suitable process known to a person skilled in the art. A person skilled in the art will know how to employ the different techniques optionally with guidance from Remington's The Science and Practice of Pharmacy (2003)

Normally, the amount of the calcium-containing compound in a tablet corresponds to from about 100 to about 1000 mg Ca such as, e.g., from about 150 to about 800 mg, from about 200 to about 700 mg, from about 200 to about 600 mg or from about 200 to about 500 mg Ca.

### Pharmaceutically acceptable excipients

in the present context, the term "pharmaceutically acceptable excipient" is intended to denote any material, which is inert in the sense that it substantially does not have any therapeutic and/or prophylactic effect per se. A pharmaceutically acceptable excipient may be added to the active drug substance with the purpose of making it possible to obtain a pharmaceutical composition, which has acceptable technical properties.

The calcium-containing compound is normally admixed with one or more pharmaceutically acceptable excipients before compression into tablets. Such excipients include those normally used in formulation of solid dosage forms such as, e.g. fillers, binders, disintegrants, lubricants, flavouring agents, colouring agents, including sweeteners, pH adjusting agents, buffering agents, stabilizing agents, etc. In the following are given examples of excipients suitable for use in a tablet prepared according to the present invention.

| Excipient | Concentration [% of formulation] |
|---|---|
| Sweetening agents | 5 - 30, if present |
| Artificial sweeteners | 0.05 - 0.3, if present |
| Flavours | 0.1 - 3, if present |
| Disintegrating agents | 0.5 - 5, if present |
| Glidant and lubricants | 0.1 - 5, if present |
| Fillers/diluents/binders | 0.1 - 30, if present |
| Film forming agents | 0.1 - 5, if present |
| Film additives | 0.05 - 5, if present |

### Sweetening agents

Examples of suitable sweeteners include dextrose, erythritol, fructose, glycerin, glucose, inositol, isomalt, lactitol, lactose, maltitol, maltose, mannitol, sorbitol, sucrose, tagatose, trehalose, xylitol, etc.. Sorbitols e.g. Neosorb P100T, Sorbidex P166B0 and Sorbogem Fines Crystalline Sorbitol available from Roquette Freres, Cerestar and SPI Polyols Inc. respectively. Maltisorb P90 (maltitol) available from Roquette Freres, Xylitol CM50, Fructofin CM (fructose) and Lactitol CM50 available from Danisco Sweeteners, Isomalt ST-PF, Gaio Tagatose and Manitol available from Palatinit, Arla Foods and Roquette, Freres respectively. Sorbitol has a sweetening effect (compared to sucrose) of 0.55; maltitol that has a sweetening effect of ≤1; xylitol that has a sweetening effect of 1, isomalt that has a sweetening effect of <0.5, etc. The sweetening effect may be of value in connection with choosing the individual sweetening agents. Thus, if a decreased tablet weight and volume are desired, it is suitable to choose a sweetening agent having a high sweetening effect.

### Artificial sweeteners

Acesulfam potassium, alitame, aspartame, cyclamic acid, cyclamate salt (e.g. calcium cyclamate, sodium cyclamate), neohesperidine dihydrochalcone, neohesperidine hydrochloride, saccharin, saccharin salt (e.g. ammonium saccharin, calcium saccharin, potassium saccharin, sodium saccharin), sucralose, taumatin and mixtures thereof.

### Flavours

Apricot, Lemon, Lemon/Lime, Lime, Orange, Mandarine, such as Apricot 501.110 AP0551, Lemon 501.051 TP0551, Lemon 501.162 AP0551, Lemon/Lime 501.053 TP0551, Lime 501.054 TP0551, Orange 501.071 AP0551, Orange TP0551, Orange 501.434 P0551, Mandarine 501.AP0551, Lemon Durarome 501.282 TDI1091 available from Firmenich, Kerpen, Germany or Juicy Lemon Flavouring T3602 available from TasteTech, Bristol, England or Lemon Lime Flavour Permseal 11029-31, Lemon Flavour Permaseal 12028-31, Lemon Flavour Ultradseal 96918-71 Available from Givaudan Schweiz AG, Kemptthal, Schweiz or Lemon Flavour Powder 605786, Lemon Flavour Powder 605897 available from Frey + Lau Gmbh, Henstedt-Ulzburg, Germany

### Disintegrating agents

Alginic acid - alginates, carboxymethylcellulose calcium, carboxymethylcellulose sodium, crospovidone, hydroxypropylcellulose, hydroxypropylmethylcellulose (HPMC), cellulose derivatives such as low-substituted hydroxypropylcellulose (e.g LH 11, LH 20, LH 21, LH 22, LH 30, LH 31, LH 32 available from Shin-Etsu Chemical Co.) and microcrystalline cellulose, polacrilin potassium or sodium, polyacrylic acid, polycarbofil, polyethylene glycol, polyvinylacetate, polyvinylpyrrolidone (e.g. Polyvidon® CL, Polyvidon® CL-M, Kollidon® CL, Polyplasdone® XL, Polyplasdone® XL-10); sodium carboxymethyl starch (e.g. Primogel® and Explotab®), sodium croscarmellose (i.e. crosslinked carboxymethylcellulose sodium salt; e.g. Ac-Di-Sol®), sodium starch glycolate, starches (e.g potato starch, maize starch, rice starch), pre-gelatinised starch.

Those skilled in the art will appreciate that it is desirable for compressible tablets to disintegrate within 30 minutes, more desirable within 15min, most desirable within 5 min; therefore, the disintegrant used preferably results in the disintegration of the tablet within 30 minutes, more preferable within 15 min, most preferable within 5 min. However, for tablets solely meant for chewing, a somewhat longer disintegration time is allowed.

Effervescent agent (e.g. mixture of sodium hydrogen carbonate (carbonates, alkaline, alkaline earth metals) and citric acid (tartaric acid, fumaric acid etc.)).

### Glidants and lubricants

Glidants and lubricants may be incorporated such as stearic acid, metallic stearates, talc, waxes and glycerides with high melting temperatures, hydrogenated vegetabable oils, colloidal silica, sodium stearyl fumarate, polyethylenglycols and alkyl sulphates.

Suitable lubricants include talc, magnesium stearate, calcium stearate, stearic acid, hydrogenated vegetable oils and the like. Preferably, magnesium stearate is used.

### Fillers/diluents/binders

Dextrins, maltodextrins (e.g. Lodex® 5 and Lodex® 10), dextrose, fructose, glucose, inositol, erythritol, isomalt, lactitol, lactose (e.g., spray-dried lactose, α-lactose, β-lactose, Tabletose®, various grades of Pharmatose®, Microtose or Fast-Floc®), maltitol, maltose, mannitol, sorbitol, sucrose, tagatose, trehalose, xylitol, low-substituted hydroxypropylcellulose (e.g LH 11, LH 20, LH 21, LH 22, LH 30, LH 31, LH 32 available from Shin-Etsu Chemical Co.), microcrystalline cellulose (e.g., various grades of Avicel®, such as Avicel® PH101, Avicel® PH102 or Avicel® PH105, Elcema® P100, Emcocel®, Vivacel®, Ming Tai® and Solka-Floc®), starches or modified starches (e.g potato starch, maize starch, rice starch, pre-gelatinised starch), polyvinylpyrrolidone, polyvinylpyrrolidone/vinyl acetate copolymer, agar (e.g. sodium alginate), calcium hydrogen phosphate, calcium phosphate (e.g. basic calcium phosphate, calcium hydrogen phosphate), calcium sulphate, carboxyalkylcellulose, dextrates, dibasic calcium phosphate, gelatine, gummi arabicum, hydroxypropyl cellulose, hydroxypropylmethylcellulose, magnesium carbonate, magnesium chloride, methylcellulose, polyethylene glycol, polyethylene oxide, polysaccharides e.g. dextran, soy polysaccharide, sodium carbonate, sodium chloride, sodium phosphate.

### Surfactants/enhancers

Surfactants may be employed such as Non-ionic (e.g., polysorbate 20, polysorbate 21, polysorbate 40, polysorbate 60, polysorbate 61, polysorbate 65, polysorbate 80, polysorbate 81, polysorbate 85, polysorbate 120, sorbitan monoisostearate, sorbitan monolaurate, sorbitan monopalmitate, sorbitan monostearate, sorbitan monooleate, sorbitan sesquioleate, sorbitan trioleate, glyceryl monooleate and polyvinylalkohol),
anionic (e.g., docusate sodium and sodium lauryl sulphate)
cationic (e.g., benzalkonium chloride, benzethonium chloride and cetrimide).

Fatty acids, fatty alcohols and fatty esters, for example:
ethyl oleate, sodium oleate, lauric acid, methyl laurate, oleic acid, sodium caprate

Dioctyl calcium sulfosuccinate, dioctyl potassium sulfosuccinate, dodecyltrimethylammonium bromide, hexadecyltrimethylammonium bromide, trimethyltetradecylammonium bromide, polyoxyehtylene ethers (polyoxyehtylene-9-lauryl ether), sodium dodecyl sulphate, sodium dioctyl sulfosuccinate, sodium laurate, sodium 5-methoxysalicylate, sodium salicylate;
bile salts, for example:
sodium deoxycholate, deoxycholic acid, sodium cholate, cholic acid, sodium glycocholate, sodium glycodeoxycholate, sodium taurocholate, sodium taurodeoxycholate;
cytoadhesives, for example:
lectins (e.g. Lycopersicon Esculentum Agglutinin, Wheat Germ Agglutinin, Urtica Dioica Agglutinin).

N-acylated amino acids (especially N-[8-(2-hydroxy-4-methoxy)benzoyl]amino caprylic acid (4-MOAC), 4-[4-(2-hydroxybenzoyl)amino]butyric acid, sodium N-[8-(2-hydroxybenzoyl)amino]-caprylate);
phospholipids, for example:
hexadecylphosphocholine, dimyristoylphosphatidylglycerol, lysophosphatidylglycerol, phosphatidylinositol, 1,2-di(2,4-octadecadienoyl)-*sn*-glycerol-3-phosphorylcholine and phosphatidylcholines (e.g. didecanoyl-L-phosphatidylcholine, dilauroylphosphatidylcholine, dipalmitoylphosphatidylcholine, distearoylphosphatidylcholine), lysophosphatidylcholine is of particular interest;
cyclodextrins, for example:
β-cyclodextrin, dimethyl-β-cyclodextrin, γ-cyclodextrin, hydroxypropyl β-cyclodextrin, methyl cyclodextrin; especially dimethyl-β-cyclodextrin is of particular interest;
fusidic acid derivatives, for example:
sodium taurodihydrofusidate, sodium glycodihydrofusidate, sodium phosphate-dihydrofusidate; especially sodium taurodihydrofusidate is of particulare interest;
others:
sodium salts of e.g. glycyrrhizic acid, capric acid, alkanes (e.g. azacycloalkanes), amines and amides (e.g. N-methyl-pyrrolidone, Azone), amino acids and modified amino acids compounds (e.g. acetyl-L-cysteine), polyols (e.g. propyleneglycol, hydrogels), sulfoxides (e.g. dimethylsulfoxide), terpenes (e.g. carvone), ammonium glycyrrizinate, hyluronic acid, isopropyl myristate, n-lauryl-beta-D-maltopyranoside, saponins, DL-octanonylcamitine chloride, palmitoyl-DL-carnitine chloride, DL-stearoylcarnitine chloride, acylcarnitines, ethylenediaminedihydro-chloride, phosphate-dihydrofusidate, sodium CAP); especially n-lauryl-beta-D-maltopyranoside is of particular interest, alpha 1000 peptide, peptide MW<1000 comprising at least 6 mol% of aspartatic- and glutamic Acid, decomposed royal jelly, prebiotica, butyrate, butyric acid, vitamin D₂, vitamin D₃, hydroxy-vitamin D₃, 1.25-dihydroxy-vitamin D₃, spirulina, proteoglycan, soyahydrolysate, lysin, lactic acid, di-fructose-anhydrid, vylitol Ca-(lactate), hydrolyzate of casein in particular a caseinoglycomacropeptide, negative ionization of CaCO₃, acetylsalicylic acid, vitamin K, creatin.

### Film forming agents

The dosage form may be provided with a coating. Hydrofilic film formers such as hydroxypropylmethylcellulose (HPMC) (e.g. HPMC E5, HPMC E15), hydroxyethylcellulose, hydroxypropylcellulose, polydextrose and maltodextrin, Sepifilm^{™} and Sepifilm^{™} LP available from Seppic S.A., Pharmacoat® available from Shin-Etsu Chemical Co, Opadry® and Opagloss® available from Colorcon and Kolicoat® available from BASF AG.

### Film additives

Acetylated monoglyceride, acetyltributyl, acetyltributyl citrate, acetyltriethyl citrate, benzyl benzoate, calcium stearate, castor oil, cetanol, chlorebutanol, colloidal silica dioxide, dibutyl phthalate, dibutyl sebacate, diethyl oxalate, diethyl malate, diethyl maleate, diethyl malonate, diethyl fumarate, diethyl phthalate, diethyl sebacate, diethyl succinate, dimethylphthalate, dioctyl phthalate, glycerin, glyceroltributyrate, glyceroltriacetate, glyceryl behanate, glyceryl monostearate, hydrogenated vegetable oil, lecithin, leucine, magnesium silicate, magnesium stearate, polyethylene glycol, propylene, glycol, polysorbate, silicone, stearic acid, talc, titanium dioxide, triacetin, tributyl citrate, triethyl citrate, zinc stearate, wax.

The following non-limiting examples are meant to illustrate the present invention.

### Examples

The trials described in the examples of the invention were carried out on a pilot model continuous fluid bed WT4/13 at the Heinen Technology Centre in Varel, Germany and on a pilot model continuous fluid bed GF 20 at the Glatt test facility in Weimar, Germany during five separate trial periods in 2003 and 2004.

### Materials and method

A preblended mixture of calcium carbonate and sorbitol containing 74.5% calcium carbonate and 23.3% sorbitol were used for all the trials described in the examples below. The composition including the amount of polyvinylpyrrolidone (PVP) was kept constant throughout the trials except for a few examples in the first series of batches. The nominal or correct amount of PVP in the calcium-containing granulate was 2.2% and the aim was to keep this constant due to regulatory difficulties, which follows a change of product composition.

Deionised water free from bacteria was used in order to prepare the granulation liquid. Granulation liquids containing 10, 15, 20, 26 and 33% polyvinylpyrrolidone (PVP) were used for the trials.

### Example 1 (batch 1-15)

The trials had the following objectives:
- To investigate whether it is possible to produce granulates of a calcium-containing compound that have essentially the same product characteristics as a particulate material, which is produced on a batch fluid bed.
- To investigate the processing windows for the critical process parameters in the continuous fluid bed WT 4/13.

A 2³ factorial design with two centre points (H2901-1 and -2) was used according to the table below where the ten individual trials in the factorial design are shown as the shaded area in the table. Three additional sets of values were also included into the model (H2801-1, -2 and -3):

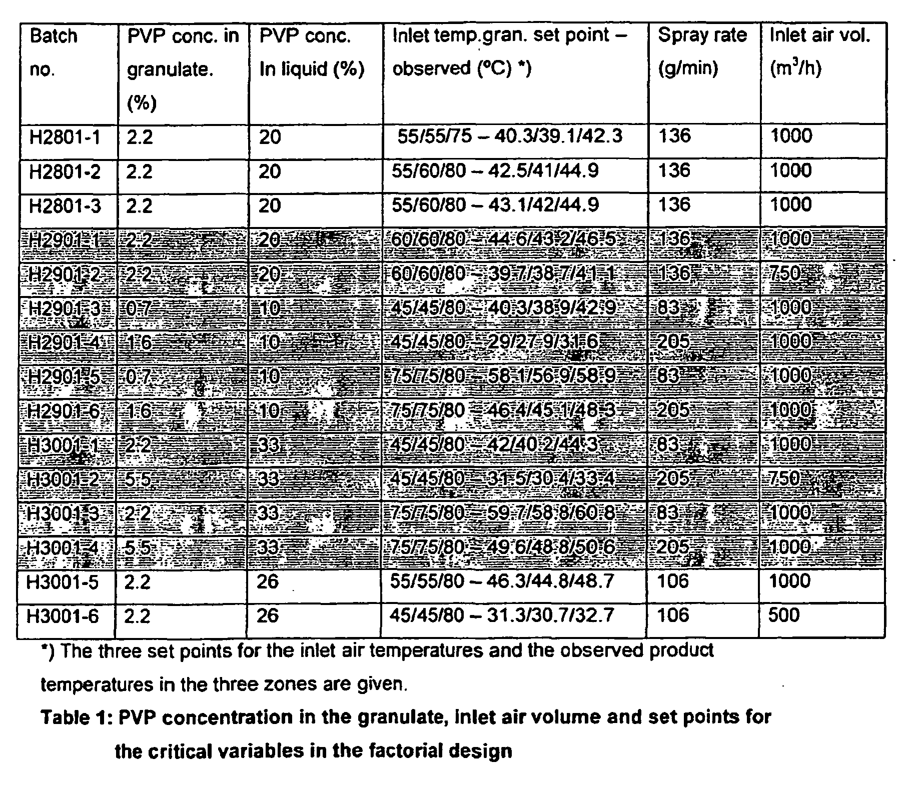

The three processing parameters to be varied with a high and low value according to the design were the PVP concentration, granulation temperature (i.e. the temperature in the first two inlet air compartments) and the spray rate.

Relatively wide processing windows were chosen in order to achieve detectable differences for the response variables. The response variables were the moisture content, particle size/ distribution and bulk density. These are the important granule characteristics for the subsequent blending and tableting properties.

The aim was to obtain a particulate material with the following limits:

| | |
|---|---|
| Particle size distribution: | > 420 µm: max 30% |
| | < 125 µm: max 50% |
| Bulk density: | 0.6 - 1.0 g/ml |
| Loss on drying: | max 0.5% |

The in-process requirement for the moisture content is set to a maximum of 0.35% due to problems, which occasionally arises due to local over wetting in the fluid bed.
This problem is probably due to a poor fluidisation in the batch fluid bed with resultant sticking of the granulate to the walls of the product container.

Generally the trials went very well in spite of the wide processing windows, which had been chosen for the critical process parameters. A satisfactory fluidisation was seen in nearly all the trials, which indicate a very robust process.

The particle size/distribution, bulk density and moisture content were measured after each trial. Generally a finer granulate was produced with a raspberry shaped surface as opposed to the more round shaped granules from a vertical fluid bed. The granules from the horizontal fluid bed thus create a more expanded bed with a resultant decrease in the bulk density.

A recurrent experience with a too moist granulate and the formation of moist lumps was seen when the spray rate was increased or when the air fluidisation volume was reduced for batches 1-15 in example 1.
This observation was thought to be due to an insufficient distribution of the granulation liquid which again increased the local over wetting of the granulate.

Visual inspection inside the processing train of the horizontal fluid bed did not reveal any deposits on the walls, bottom screen or on the nozzles. When opening the hinged bottom screen there was no unusual accumulation of powder beneath the screen. Thus the present problem with sticking and clogging of the granulate inside the existing vertical batch fluid bed and the lodging of granulate beneath the product screen seemed to be much less pronounced in a horizontal continuous fluid bed.

### Results

5 to 6 kg samples of granulates from the above trials were collected and the granulates were sieved on a 2 mm sieve and the weight noted. The moisture content of the oversize fraction was measured as well as the moisture content in the sieved fraction below 2 mm. The bulk density of the sieved fraction was measured and the particle size/distribution by sieve analysis was repeated. Particle size and distribution was additionally measured by Malvern analysis.

The results are given in the table below.

**Table 2: Granulate characteristics from example 1 and batch 1 to 15**

| Batch no. | Moisture (%age) | Moisture >2mm (%age) | Bulk Density (g/ml) | Sieve analysis (%age) | | | | Malvern analysis (µm) | | |
|---|---|---|---|---|---|---|---|---|---|---|
| | | | | >2 mm | >425 µm | 425-125 µm | <125 µm | D₁₀ | D₅₀ | D₉₀ |
| H2801-1 | 0.10 | 0.20 | 0.65 | 1.7 | 1.8 | 55.9 | 42.3 | 30.0 | 150.0 | 335.2 |
| H2801-2 | 0.30 | 0.30 | 0.70 | 1.7 | 1.8 | 50.9 | 47.3 | 24.3 | 133.6 | 318.7 |
| H2801-3 | 0.30 | 0.30 | 0.71 | 2.0 | 2.0 | 49.2 | 49.1 | 29.5 | 155.3 | 367.2 |
| H2901-1 | 0.20 | 0.20 | 0.68 | 3.0 | 2.3 | 45.3 | 53.0 | 25.9 | 127.8 | 305.5 |
| H2901-2 | 0.30 | 0.30 | 0.67 | 3.6 | 2.3 | 46.9 | 51.7 | 26.3 | 132.4 | 319.0 |
| H2901-3 | 0.20 | 0.20 | 0.73 | 0.5 | 1.3 | 28.6 | 70.7 | 19.8 | 113.8 | 336.8 |
| H2901-4 | 0.30 | 0.20 | 0.62 | 0.4 | 5.0 | 47.9 | 48.2 | 25.7 | 128.4 | 364.2 |
| H2901-5 | 0.10 | 0.20 | 0.78 | 1.0 | 1.7 | 30.1 | 68.3 | 18.5 | 120.3 | 340.5 |
| H2901-6 | 0.20 | 0.20 | 0.71 | 6.3 | 5.3 | 59.4 | 35.9 | 29.3 | 154.5 | 396.0 |
| H3001-1 | 0.30 | 0.40 | 0.78 | 0 | 1.0 | 21.5 | 78.0 | 16.7 | 87.0 | 226.6 |
| H3001-2 | 0.40 | 0.50 | 0.64 | 4.9 | 5.8 | 60.0 | 34.8 | 47.6 | 219.3 | 536.9 |
| H3001-3 | 0.20 | 0.20 | 0.82 | 0.3 | 1.3 | 30.2 | 68.9 | 18.9 | 102.0 | 305.7 |
| H3001-4 | 0.30 | 0.30 | 0.69 | 3.9 | 5.3 | 60.6 | 34.9 | 38.1 | 182.6 | 429.8 |
| H3001-5 | 0.20 | 0.30 | 0.78 | 0.2 | 1.7 | 33.5 | 66.0 | 22.2 | 120.0 | 326.7 |
| H3001-6 | 0.30 | 0.30 | 0.66 | 0.9 | 0.9 | 39.1 | 61.2 | 22.7 | 116.4 | 289.1 |

Moisture content: The results for the moisture content show values well below the in process requirement of maximum 0.35%. Only one granulate is over wetted (H3001-2) and the reason being that this batch received the highest spray load at the lowest temperature with a PVP concentration of 33% in the granulation liquid.

Bulk density: The mean value for the bulk density was 0.71 g/ml. This is about 15% less than that obtained from a batch fluid bed with the same composition for the granulate employed in the two cases. The reason is that the granulate from the continuous fluid bed has been less subjected to frictional forces and thus show a more expanded structure with a resultant low value for the bulk density.

Particle size/distribution: The results from the sieve analysis show granulates that are generally too fine where 8 of the batches are out of specification with respect to the fine size fraction below 125 µm. The Malvern results also confirm this where the mean particle size for the 15 granulates is 136 µm. This is somewhat lower when compared to the mean particle size from a batch fluid bed, which is in the range of 200 - 250 µm.

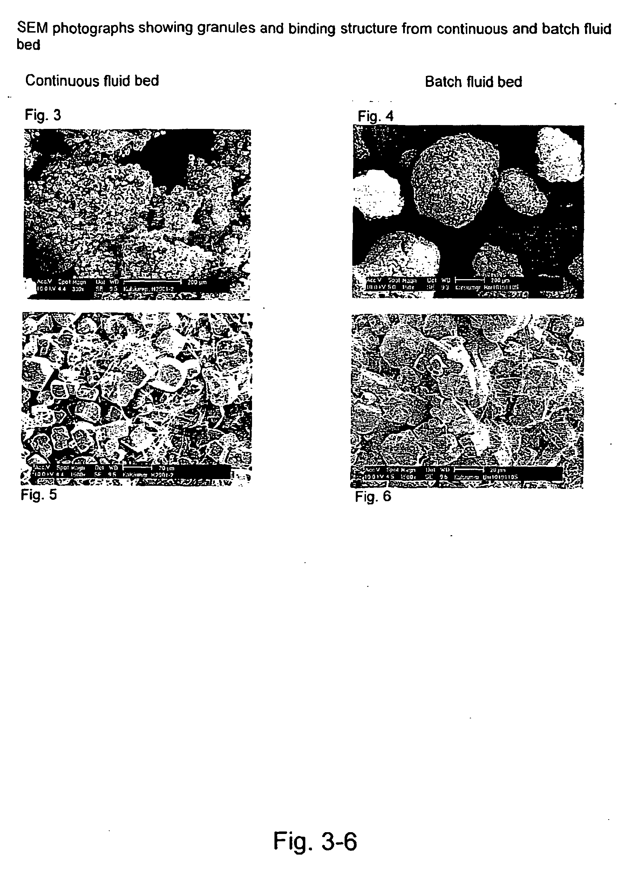

SEM photographs: Scanning electron microscope pictures are shown in Figures 3-6 for the Heinen batch H2901-2 and for a fluid bed granulate based on a batch fluid bed.

The granules from the continuous fluid bed process are characteristically more irregular in shape as these have not been exposed to the same friction and gravitational forces that are present in a batch process. The two pictures with the largest magnification show that the binding mechanism is the same between the two technologies. Here the fine strands of PVP can be seen to bind the cubic shaped crystals of calcium carbonate together in an interlocking mesh. This homogenous distribution of the binder also explains the excellent consolidation properties of the granulate during tablet compression.

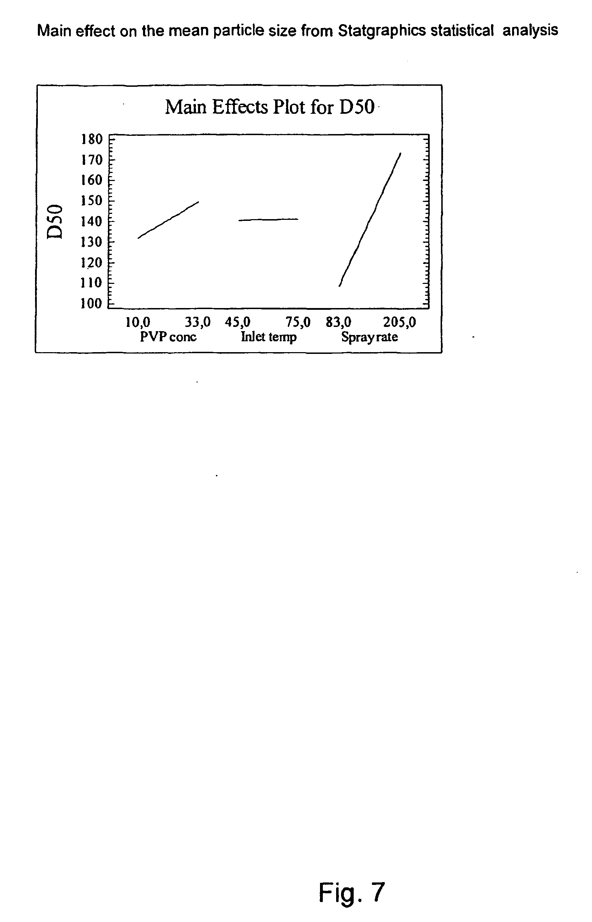

Statistical analysis and the significance of spray rate and PVP concentration is depicted in figure 7, which shows the main effects on the mean particle size. The three variables investigated in the factorial design were PVP concentration, inlet air temperature and spray rate. It is seen that the most important parameter with respect to particle size is the spray rate. The PVP concentration has a positive effect on the particle size but it is far less pronounced than the effect of the spray rate. The temperature has no effect on the particle size.

The latter observation is surprising, as one would have expected an increase of the inlet temperature to cause an increase in the evaporation rate resulting in a decreased agglomeration capacity. It means that the inlet temperature can be set to a high level in order to optimize the drying capacity of the continuous process.

Thus, when considering the batches which have an amount of 2.2% PVP it can be seen that it is the PVP concentration of 20% which gives the most favourable mean particle size. From the results it was concluded that a more diluted PVP concentration or a higher spray load with the correct amount of 2.2% PVP would probably lead to a further increase of the mean particle size and possibly that a higher inlet granulation temperature could be used. This was investigated during the trials in example 3 in the following text.

The main effect seen from the trials in example 1 was the importance of the spray load where an increase in the spray load results in an increased mean particle size.

### Sensoric evaluation

A sensoric evaluation was carried out by a panel of 7 qualified persons. Batch PU30305 with a low bulk density and PU30306 with a high bulk density from the constituent Heinen calcium granulate was tested against a reference based on the fluid bed batch wise calcium granulate (PU30307). The two batches based on the Heinen granulates were tested against the reference by a paired test with respect to orange flavour and hardness.

The sensoric panel did not detect any significant difference at a 5% level between the samples in each case.

### Example 2 (batch 16-21)

The trials carried out at the Glatt facility in Dresden had the following objectives:
- To investigate to agglomeration and drying in the Glatt continuous fluid bed GF 20 with bottom spray.

### Results

The sampling and methods of analysis were carried out in the same fashion as for example 1.

The results for the granulates produced are given in the table below:

**Table 3: Granulate characteristics for granulates produced with Glatt GF 20**

| Batch no. | Moistture | Bulk density | Sieve analysis* - G/NP | | | Malvern analysis (µm) | | | |
|---|---|---|---|---|---|---|---|---|---|
| | | | >450µm | 450-125µm | <125µm | D₁₀ | D₅₀ | D₉₀ | Span value |
| Test 2, Gr.2 | 0.2 | 0.80 | 0.9 | 30.2 | 68.9 | 22.7 | 126.2 | 288.0 | 2.10 |
| Test 3, Gr. 3 | 0.2 | 0.80 | 1.8 | 33 | 65.7 | 22.5 | 127.5 | 285.5 | 2.16 |
| Test 3, Gr. 7 | 0.2 | 0.77 | 1.8 | 33 | 65.7 | 22.2 | 127.7 | 279.4 | 2.01 |
| Test 3, Gr. 10 | 0.2 | 0.85 | 2.1 | 32.6 | 65.8 | 17.8 | 123.1 | 280.7 | 2.14 |
| Test 4, Gr. 7 | 0.2 | 0.78 | 3.1 | 38.5 | 58.9 | 22.7 | 142.9 | 329.6 | 2.15 |

Moisture content and bulk density: The results for both the moisture content and bulk density show a very good reproducibility with values well inside the specified requirements. The values for the bulk densities are higher than the results from the Heinen trials. This is most likely because the particle size of the granulates from the Glatt trials are finer with a resultant denser packing tendency for granulates.

Particle size/distribution: There is a good correlation between the Malvern results and the sieve analysis. The value for the mean particle size varies very little except for the last batch where the mean value is increased from approximately 125 µm to 143 µm. The size fraction between 450 and 125 µm is too small and the size fraction below 125 µm is too big. The reason for the relatively small value for the mean particle size was that the process was carried out with processing conditions, which favoured "dry conditions". Further optimisation including increasing the spray load would have been necessary in order to increase the value for the mean particle size.

Bottom spray: The trials showed that bottom spray could be effectively used to produce granulates, which was the aim for this set of trials.

### Example 3 (batch 22-27)

**These trials had the following objectives:**
- Concentration of PVP in the spraying liquid (15 and 20%) with an adjustment of spraying rate.
- Number and type of nozzles: changed from two to three nozzles. The three nozzles were positioned after each in the middle of the bed where the first nozzle sprayed at an angle against the direction of the moving bed and the two next nozzles at an angle with the moving bed. Two nozzles were positioned in the first zone whereas the third nozzle was positioned in the second zone.
- Inlet air temperature

**Table 4: PVP concentration in granulation liquid and critical process variables for the trials carried out on a Heinen WT4/13**

| Batch no. | PVP conc. In liquid (%) | Inlet temp.gran. set point -observed product temp.s (°C) | Spray rate (g/min) | Inlet air vol. (m³/h) |
|---|---|---|---|---|
| 1A 12:15 | 20 | 60/60/80 - 45.3/45.7/46.9 | 141 | 1000 |
| 2A 12:40 | 20 | 60/60/80 - 45.2/45.9/46.9 | 141 | 1000 |
| 4A 15:10 | 15 | 80/80/80 - 51.7/51.7/53.0 | 187.5 | 1000 |
| 5A 15:50 | 15 | 80/80/80 - 51.7/51.7/53.0 | 187.5 | 1000 |
| 6A 16:30 | 15 | 80/80/80 - 51.7/51.7/53.0 | 187.5 | 1000 |

The trials were carried out with an inlet air humidity of 8 g H₂O/kg air as opposed to the inlet air humidity for the batches in example 1, which was 4 g H₂O/kg air. This difference caused an increase in the relative humidity inside the granulation processing compartments, which again increased the agglomeration capacity when compared to the conditions during the trials reported in Example 1.

All the five batches were dried for an additional 10 min at 80°C in a laboratory batch fluid bed (mobatch) to compensate for a too short drying zone in the WT 4/13.
The two first trials with 20% PVP and with an inlet granulation temperature of 60°C resulted in a too high moisture content. The inlet temperature was thus increased to 80°C for the subsequent trials with 15% PVP.

### Results

The granulates were analysed according to the same procedure as for the trials carried out in week 5. The results are given in the below table:

**Table 5: Granulate characteristics for trials from example 3**

| Batch no. | Moisture | Bulk density | Sieve analysis | | | | Malvern analysis (µm) | | | |
|---|---|---|---|---|---|---|---|---|---|---|
| | | | >2 mm | >425 µm | 425-125 µm | <125 µm | D₁₀ | D₅₀ | D₉₀ | Span value |
| 1A 12:15 | 0.28 | 0.69 | 1.4 | 9.2 | 67.7 | 22.9 | 52 | 185 | 424 | 2.01 |
| 2A 12:40 | 0.25 | 0.74 | 0.9 | 6.2 | 59.7 | 34.1 | 36 | 174 | 409 | 2.14 |
| 4A 15:10 | .29 | 0.72 | 1.9 | 8.2 | 72.1 | 19.9 | 79 | 232 | 476 | 1.71 |
| 5A 15:50 | 0.31 | 0.72 | 1.3 | 8.5 | 71.5 | 20.1 | 60 | 225 | 449 | 1.72 |
| 6A 16:30 | 0.29 | 0.73 | 0.7 | 7.3 | 72.3 | 20.6 | 58 | 224 | 446 | 1.73 |

Moisture content and bulk density: The results for the moisture measurements and bulk density were all inside the requirements and very similar to the results achieved from the trials in example 1.

Particle size/distribution: Batch 1A and 2A are produced with the same set points for the processing parameters as H2901-1 and H2901-2 (table 1 and 2), yet there is a significant difference in the particle size between the two pairs of batches. The particle size given as D₁₀, D₅₀ and D₉₀ are 26, 130, 312 µm for batch H2901-1/H2901-2 and 44, 180, 417µm for batch 1A/2A. In addition to an increase in the particle size for the batches produced in example 3, there is also a change in the particle size distribution where the distribution has become narrower for the batches produced in example 3.
The big difference in the inlet air moisture content explains the difference in the particle size between the granulates produced during the two test periods.

The product temperatures for the batches run at 60/60/80°C in example 1 and in the present example are 45.8 and 40.8 °C, respectively, which are equivalent to relative moisture contents in the processing chamber of 16 and 29%.

Thus there is nearly a twofold increase in the relative moisture, which have obviously influenced the agglomeration capacity

A comparison between the batch fluid bed process and the continuous fluid bed process was carried out with respect to the particle size distribution.

The results from the Malvern analysis are given in the table below.

**Table 6: Mean particle size, distribution and span value for granulates from batch fluid bed compared to the same characteristics from three series of Heinen batches**

| Fluid bed batches | Malvern analysis (µm) | | | |
|---|---|---|---|---|
| | D₁₀ | D₅₀ | D₉₀ | Span value |
| 10 FB batches (Aeromatic S6, batchsize: 250 kg) | 61 | 183 | 530 | 2.56 |
| 10 FB batches (GPCG 300, batchsize: 750 kg) | 42 | 225 | 637 | 2.67 |
| Example 3: Heinen CFB batches: 4A 15:10, 5A 15:50 and 6A 16:30 - (15%) | 66 | 227 | 457 | 1.72 |
| Example 4: Heinen CFB batch no. 10206383 | 60 | 246 | 516 | 1.85 |
| Example 5: Heinen CFB batch no. 31-37 | 82 | 272 | 604 | 1.92 |
| (continuous fluid bed is denoted CFB, whereas the batchwise fluid bed is denoted FB) | | | | |

The Heinen CFB granulates were produced on three different occasions and with the same set of processing conditions as specified under the respective examples. From the table it can be seen that the particle size distribution is more narrow for the CFB granulates when compared to the results from the batch fluid beds. The narrow particle size distribution for the CFB granulates is shown by the low result for the span value. A more narrow size distribution for the CFB batches is also exemplified by the lower value for D₉₀ with a reduction in the course fraction of approximately 20-25% when comparing batch series with approximately the same mean particle size.

### Control of mean particle size by optimisation of the moisture load

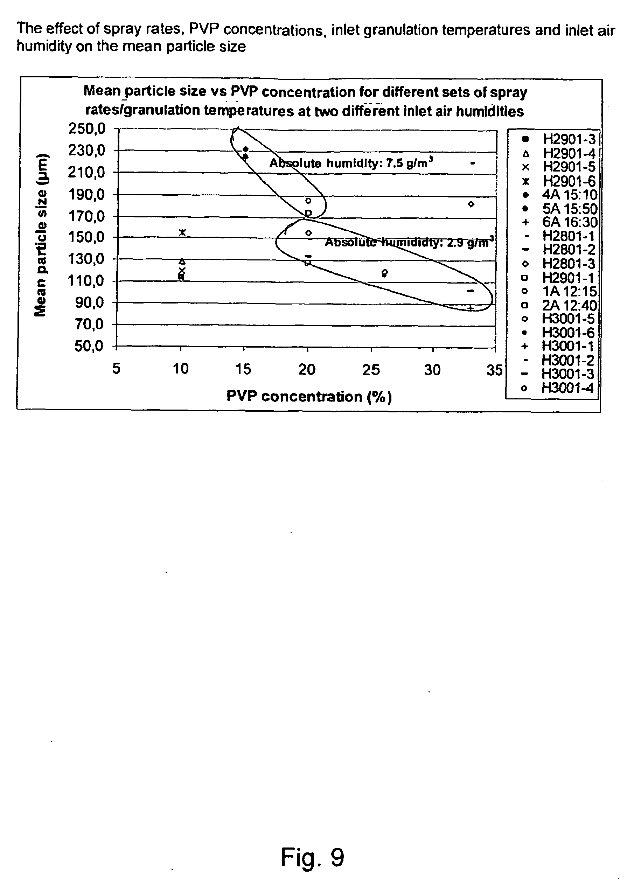

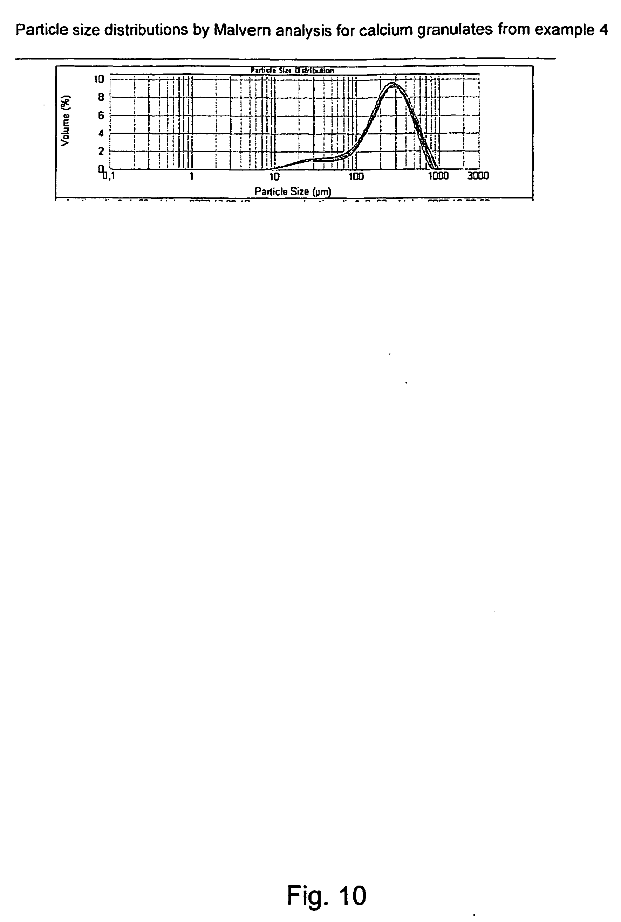

The moisture load is here defined as the combined moisture effect of the inlet air humidity and the spray load (spray rate at a certain concentration of PVP). The PVP concentration has been plotted against the mean particle size from the Malvern analysis in figure 9. The plotted results inside the two loops all contain the nominal or correct amount of 2.2% PVP whereas the PVP concentrations for the other batches are either higher or lower than this value. The experiments have been carried out at two different times in week 5/2003 and 32/2003 where the absolute moisture contents in the inlet air were 2.9 and 7.5 g/m³ respectively.

Starting with the 10% PVP batches with a spray rate of 83 g/min (H2901-3 and -5) one can see that there is little difference between the granulation temperatures of 45 and 75°C. When the spray rate is increased to 205 g/min (H2901-4 and -6) there is a wider difference between the two inlet granulation temperatures where the highest inlet temperature produces the highest value for the mean particle size.

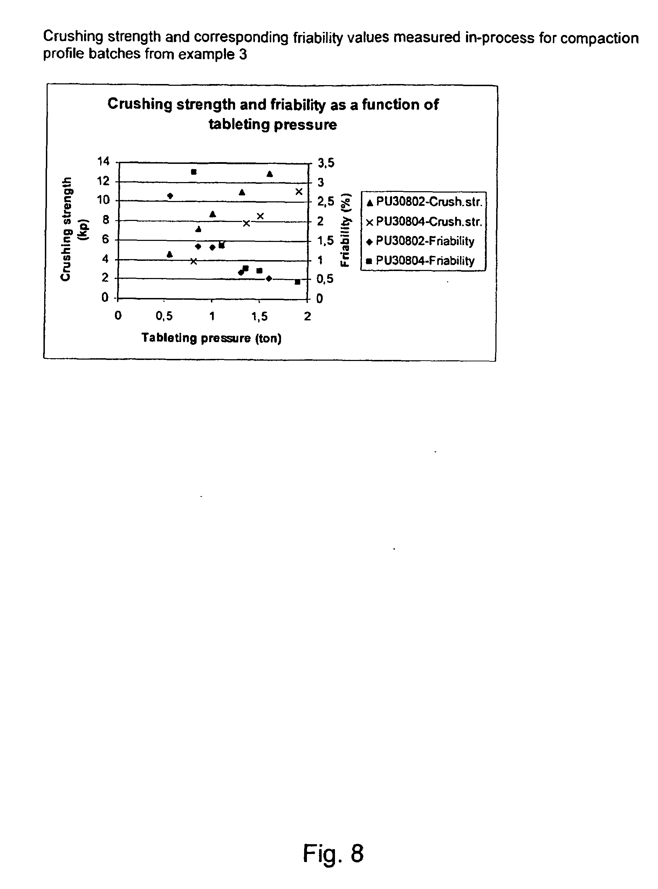

Three batches have been produced in week 32 with a PVP content of 15% and with inlet air moisture content of 7.5 g/m³. These three batches have a mean particle size of 227 µm and thus constitute the population with the largest mean particle size among the batches depicted in figure 8. This is due to the fact that these three batches have experienced the greatest moisture load.

Five batches have been produced in week 5 with a 20% PVP concentration in the granulation liquid and with a moisture content in the inlet air of 2.9 g/m³ (H2801-1,-2,-3 and H2901-1 and -2). The batches were produced with a spray rate of 136 g/min and did only differ by 5°C with respect to the inlet air temperature during the granulation phase. As seen from the graph the mean particle size for the five batches is nicely gathered in a size range from 130 to 155 µm, which indicate a satisfactory reproducibility.

Two batches were produced in week 32 with an identical composition of the granulation liquid but with a moisture content of the inlet air of 7.5 g/m³. From table 2 and 5 it can be seen that the mean particle size has increased from 140 µm to 180 µm for batches containing 20% PVP and produced in week 5 and 32 respectively. The reason for the increased mean particle size for the two batches in week 32 is the increased moisture load which these two batches have been exposed to.

Two batches (H3001-5 and -6) are produced with 26% PVP concentration in the granulation liquid and with a spray rate of 106 g/min where the granulation inlet temperature differ by 10°C between the two batches. Here the mean particle size has been reduced to approximately 120 µm.

A further reduction in the mean particle size due to a decreased spray load is seen when the PVP concentration is increased to 33% and the content of PVP is kept at the theoretical value of 2.2% (H3001-1 and -3). Spray load is here defined as the amount of moisture in the spray rate. A spray rate of 83 g/min and a PVP content of 33% are thus giving the lowest spray load among the trials in the experimental design. At this PVP concentration and spray rate it seems that the spray load is insufficient in order to achieve a satisfactory agglomeration.

The largest particle size is seen for the highest spray rate of 205 g/min but this also contain as much as 5.5% of PVP in the granulate. At this spray rate the spray load is sufficient and in combination with an increased PVP content this ensures an increased agglomeration capacity.

The results depicted in figure 9 show that it is the combined effect of the inlet air moisture content and the spray load, which determine the mean particle size of the resultant calcium granulate. From the graph it can be seen that the mean particle size can be varied in a controlled fashion between 87 and 227 µm.

### Example 4 (batch 28-30)

A long-term trial with the most favourable set points for the critical process variables was carried out in order to investigate the stability of the process and at the same time to produce enough granulate for a scale up trial on an industrial scale.

The set point for the critical process variables were as follows:
- Inlet air volume: 1000 m³/h (at approx. 35°C)
- Absolute moisture content of inlet air: 4 g/kg
- Concentration of PVP in granulation liquid: 15%
- Feed rate of powder mixture: 75 kg/h
- Retention time: 1 hour
- Spray rate with three nozzles: 187. 5 g/min
- Inlet air temperature in all three compartments: 80°C

Results from particle size distribution, bulk density and loss on drying from batch 10206383 (continuous fluid bed granulate) before and after mixing (batch 10206906) are given below.

**Table 7: Particle size distribution, bulk density and moisture content for 6 samples of continuous fluid bed calcium granulate**

| **Process stage** | **Results batch no. 10206383** | | | | | | | | |
|---|---|---|---|---|---|---|---|---|---|
| **Before mixing** | Sieve (%) | | | Malvern (µm) | | | | Bulk density (g/cm³) | Loss on drying (%) |
| | >425 µm | <425> 125µm | <125 µm | d(0,5) | d(0,1) | d(0,9) | Span value | | |
| 1. | 12.6 | 70.8 | 16.8 | 231 | 55.4 | 471 | 1.79 | 0.68 | 0.22 |
| 2. | 13.6 | 68.4 | 18.1 | 235 | 51.8 | 494 | 1.88 | 0.68 | 0.20 |
| 3. | 14.8 | 67.8 | 17.5 | 244 | 56.5 | 511 | 1.86 | 0.68 | 0.24 |
| 4. | 16.6 | 69.9 | 13.6 | 251 | 59.2 | 540 | 1.92 | 0.66 | 0.24 |
| 5. | 18.6 | 68.3 | 13.1 | 262 | 69.3 | 539 | 1.79 | 0.65 | 0.25 |
| 6. | 21.2 | 66.3 | 12.7 | 255 | 67.3 | 538 | 1.84 | 0.64 | 0.22 |

| **After mixing** | **Results batch no. 10206906 from Vrieco mixer 3** | | | | | | | | |
|---|---|---|---|---|---|---|---|---|---|
| 1. | 12.9 | 61.1 | 26.2 | 234 | 39.5 | 539 | | 0.81 | - |

Bulk density and moisture content: The bulk densities for the samples taken from the big-bags during the continuous granulation process are typically low in value. During the mixing process the bulk density is increased up to a level which is comparable to the bulk density for a granulate based on a batch process.
The results for the moisture content are low and well inside the in process requirement of 0.35%.

Particle size/distribution: The major proportion of the granulates are inside the size fraction of 425 to 125 µm where as only a small size fraction is below 125 µm. The mean particle size is only reduced from 246 to 234 µm after the completion of the mixing process which shows that the granulate mixture can withstand the mixing process in a satisfactory fashion. The reproducibility of the particle size and distribution for the six samples of granulate from the continuous granulation process is depicted in figure 7 which show the Malvern analysis for the samples.

Scale up trial: 1288 kg of tablet end mixture was produced on a Vrieco conical screw mixer and 16 mm biconvex tablets were produced on a Fette 3090 tableting machine with a tableting speed of 6000 tablets per min according to the following batch formula:

| | |
|---|---|
| Calcium granulate from continuous Heinen fluid bed : | 1242 kg |
| Flavour granulate lemon: | 37.5 kg |
| Cholecalciferol 100 CWS - sieved 250 µm: | 3.26 kg |
| Aspartame: | 0.741 kg |
| Magnesium stearate: | 4.45 kg |

The tablets were automatically transferred to a packaging machine and packed into 275 ml tablet containers.
The tableting process at the specified tableting speed of 6000 tablets per min was carried out in a satisfactory fashion without any problems. Likewise did the automatic transfer of tablets to the packaging machine and the packaging process itself proceed in a normal fashion without any breakage of tablets.

### Example 5 (batch 31-42)

The set of trials had the following objectives:
- To show reproducible results with respect to granulate characteristics in a long term trial period.
- To show process robustness with respect to a change in the quality for calcium carbonate with different physical characteristics.
- To show control of the mean particle size by varying the granulation spray load.
- To show satisfactory reproducibility for the homogeneity of vitamin D₃ in chewable calcium tablets.

The same set of processing parameters for the continuous granulation process was used as described for example 4. The granulation process was run for 13 hours in order to produce a sufficient amount of calcium granulate for the mixing trials and to show reproducibility as given in the below table.

### Results

**Table 8: Particle size distribution and bulk density for 7 samples of continuous fluid bed calcium granulate**

| Batch no. | Bulk density | Sieve analysis (%age) | | | Malvern analysis (µm) | | | |
|---|---|---|---|---|---|---|---|---|
| | | >425µm | 425-125µm | <125 µm | D₁₀ | D₅₀ | D₉₀ | Span value |
| 6/513:00 | 0.69 | 15.2 | 64.7 | 20.2 | 67.6 | 255 | 553 | 1.90 |
| 6/513:30 | 0.70 | 15.5 | 63.6 | 21.0 | 57.2 | 251 | 565 | 2.02 |
| 6/514:40 | 0.67 | 16.1 | 65.7 | 18.2 | 76.9 | 259 | 562 | 1.87 |
| 7/5 09:55 | 0.78 | 13.5 | 60.2 | 26.5 | 64.5 | 260 | 568 | 1.93 |
| 7/510:20 | 0.67 | 20.3 | 67.0 | 12.7 | 107 | 311 | 770 | 2.13 |
| 7/511:00 | 0.65 | 17.5 | 67.3 | 15.3 | 85 | 268 | 576 | 1.83 |
| 7/511:40 | 0.64 | 19.4 | 67.1 | 13.5 | 112 | 300 | 640 | 1.76 |
| Mean | 0.69 | 16.9 | 65.1 | 18.2 | 81.5 | 272 | 604 | 1.92 |
| Stand. dev. | 0.05 | 2.4 | 2.5 | 4.9 | 21.2 | 23.7 | 78.3 | 0.12 |

Reproducibility: The granulate characteristics with respect to bulk density and particle size and distribution show very reproducible and constant values. A narrow particle size distribution is again exemplified by the large proportion of granulate between 425-125 µm from the sieve analysis and from the low result for the span value from the Malvern analysis.

### Control of particle size by varying spray load

**Table 9: High and low spray load at two different inlet air granulation temperatures**

| Batch no. | PVP conc. In liquid (%) | Inlet air set points - (°C) *) | Spray load (g H₂O/min) | Comment |
|---|---|---|---|---|
| 1a | 33 | 55/55/70 | 125.6 | Low temp.-dry conditions |
| 1b | 20 | 55/55/70 | 150.0 | Low temp.-wet conditions |
| 1e | 25 | 80/80/80 | 140.6 | High temp.-dry conditions |
| 1f | 15 | 80/80/80 | 159.4 | High temp.-wet conditions |

The four batches were produced with a granulation spray rate of 187.5 g/min and two trials with a high and low spray load respectively were carried out at two inlet granulation temperatures as shown above. The four trials proceeded satisfactory giving rise to calcium granulates with characteristics as shown below.

**Table 10: Particle size distribution and bulk density for granulates with a low and high spray load**

| Batch no. | Bulk density | Sieve analysis (%age) | | | Malvern analysis (µm) | | |
|---|---|---|---|---|---|---|---|
| | | >425µm | 425-125µm | <125 µm | D₁₀ | D₅₀ | D₉₀ |
| 1a | 0.86 | 0.5 | 30.0 | 69.6 | 16.7 | 95.3 | 258 |
| 1b | 0.67 | 2.5 | 48.1 | 49.6 | 28.7 | 130 | 301 |
| 1e | 0.69 | 1.2 | 38.1 | 60.8 | 20 | 106 | 277 |
| 1f | 0.70 | 24.9 | 55.3 | 19.9 | 58.6 | 295 | 642 |

From the above results it can be seen that the particle size can be controlled by the spray load and that this control is most effectively executed at an inlet granulation temperature of 80°C. The results show a control of the mean particle size in a range of going from 100 to 300 µm.

Robustness: A trial has been carried out in order to show robustness with respect to the processability of a calcium carbonate quality with different physical characteristics. All the trials from the example series have been carried out with Scoralite 1A+1B which consists of cubic or pseudo-cubic mono-size crystals with smooth and regular surfaces. The crystals have a mean particle size of approximately 10 to 20 µm, a specific surface area in the range of 0.3 to 0.6 m²/gram and a bulk density in the range of 0.9 to 1.2 g/ml.

A new quality for calcium carbonate denoted: "Calcium carbonate precipitated, extra pure, 102064 from Merck" has a particle size in the range of 10 to 16 µm, a specific surface area of 0.3 to 0.6 m²/gram and a bulk density in the range of 0.4 to 0.7 g/ml. Scanning electron microscope pictures had revealed that the surface of the crystals were irregular where one particle consisted of three or four crystals which were "glued" together. Production trials in a 250 kg batch fluid bed had demonstrated that the existing set of set-points for the processing parameters for a batch fluid bed could not be used for this quality. A laborious upscaling study had to be initiated with a definition of a new set of set-points for the critical processing parameters for this quality. This is described in the reference example below.

Thus the aim of the study with this new quality of calcium carbonate in the Heinen continuous fluid bed WF 4/13 was to show that the same set of set-points for the processing parameters could be used when changing from the existing Scoralite 1A+1B to the new Merck quality without any difficulties related to processability or unsatisfactory granulate characteristics.

The change of raw material qualities was carried out without interrupting the infeed or the set-points for process parameters. A small reduction in the feed rate was noted due to the reduced bulk density of the Merck quality, which affected the volumetric dosing of the raw material preblend.

The trial with Merck was run for four and half hours without changing any of the process parameters.

The granulate characteristics were well inside the requirements as shown in the below table:

**Table 11: Granulate characteristics for calcium granulate with Merck 102064**

| Batch no. | Bulk density | Sieve analysis (%age) | | | Malvern analysis (µm) | | | |
|---|---|---|---|---|---|---|---|---|
| | | >425µm | 425-125µm | <125µm | D₁₀ | D₅₀ | D₉₀ | Span value |
| 6/5 18:30 | 0.63 | 4.8 | 58.5 | 36.9 | 36 | 172 | 407 | 2.15 |

The fact that the process was run without changing any of the process parameters and with granulate characteristics well inside the requirements show that the continuous fluid bed process is a robust process able to tolerate changes in the physical characteristics of the raw material in a satisfactory fashion. This is in contrast to a batch fluid bed process where an extensive qualification set of trials was necessary in order to implement the new quality of calcium carbonate as described in the reference example.

Homogeneity of vitamin D₃: The calcium granulate was used in order to produce three mixing batches each containing 230 kg of granulate. The mean particle sizes before mixing for the three batches were 217, 203 and 252 µm respectively.

Three batches of calcium chewable tablets containing 10 µg (400 I.U.) of vitamin D3 and with lemon flavour were produced with the same composition as in example 4.

The Ruberg 400 mixer is a twin shaft vertical low shear mixer with forced convection and with an effective mixing volume of 320 litres (80%). The batch size for the three batches was 238.52 kg. All the ingredients except for magnesium stearate were charged into the mixer and blended for 4 min at 50 RPM, magnesium stearate was added and mixed for an additional 1 min. Three 3 kg samples were taken as samples during the discharge of the mixer: At the start of the discharge, in the middle and the end of discharge. The three separate samples for each batch were made into 16 mm normal convex shaped tablets and a representative sample of 10 tablets taken out for the three lots of tablets to be analyzed with respect to the content of vitamin D₃. The results for the content uniformity with respect to vitamin D₃ as IU (international units) for the three batches of calcium chewable tablets are shown in the below table:

**Table 12: Uniformity of content for vitamin D₃ for three batches of calcium chewable tablets with 20 µg vitamin D₃**

| Tablet | PU40101 | | PU40102 | | PU40103 | |
|---|---|---|---|---|---|---|
| | IU | % | IU | % | IU | % |
| 1 | 486 | 103.2 | 486 | 101.9 | 482 | 98.7 |
| 2 | 468 | 99.3 | 474 | 99.3 | 488 | 100.0 |
| 3 | 463 | 98.3 | 470 | 98.6 | 485 | 99.2 |
| 4 | 469 | 99.4 | 476 | 99.7 | 469 | 96.0 |
| 5 | 487 | 103.3 | 484 | 101.4 | 506 | 103.6 |
| 6 | 468 | 99.4 | 497 | 104.2 | 507 | 104.7 |
| 7 | 502 | 106.4 | 446 | 93.4 | 512 | 104.7 |
| 8 | 447 | 94.9 | 479 | 100.4 | 496 | 101.4 |
| 9 | 468 | 99.3 | 492 | 103.2 | 467 | 95.6 |
| 10 | 455 | 96.5 | 467 | 98.0 | 474 | 97.1 |
| Average | 471 | | 477 | | 488 | |
| SD | 16.1 | | 14.6 | | 16.1 | |
| | | | | | | |
| RSD | 3.4 | | 3.1 | | 3.3 | |

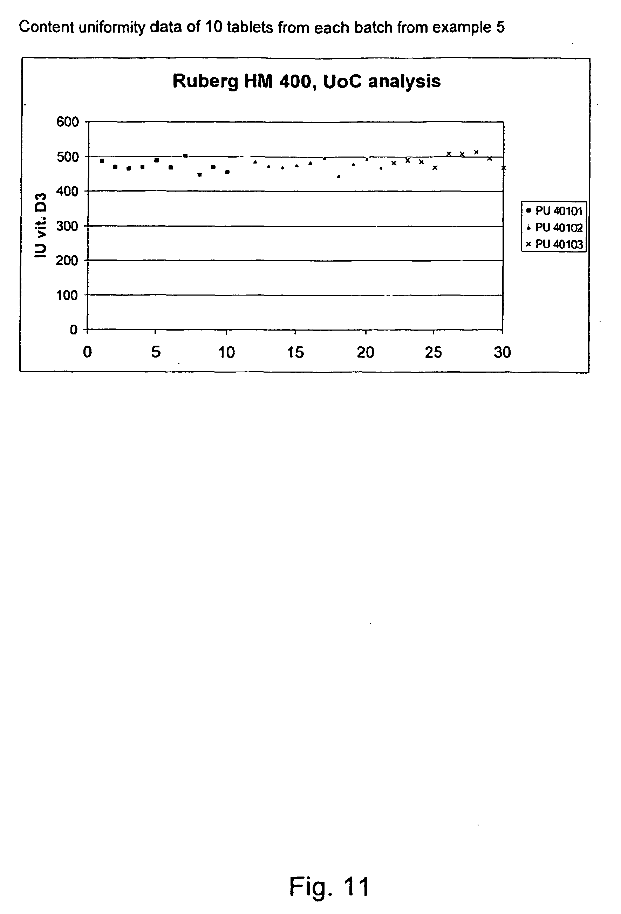

The results show an excellent homogeneity of vitamin D₃ in the tablets where all the values are well inside the requirements of ±155% of the average content of vitamin D₃. This is depicted in figure 11 where the content of cholecalciferol is plotted for the three batches. The good homogeneity is also characterised by the low values for the relative standard deviation in the range of 3.1 - 3.4%.

The two most important considerations concerning the particle size and distribution of the calcium granulate are related to the homogeneity of the distribution of vitamin D₃ and the tableting properties of the final tableting mixture.

At present the mean particle size of the sieved vitamin D₃ is in the region of 180-200 µm and the aim is to get the mean particle size reduced to approximately 150-180 µm in order to improve the homogeneity of vitamin D₃ even further. The decrease in the particle size for vitamin D₃ is beneficial because of the resultant increase in the number of vitamin D₃ particles per dosage unit, which ensures a more even distribution of vitamin D₃.

In this respect the Heinen granulates are well suited to match the particle size and distribution of the vitamin D₃.

Segregation problems in the final tableting mixture may occur when the coarse size fraction in the tableting mixture increases. This may occur as a function of vibration during handling or uneven flow in the IBC containers during the tableting step. In this respect it would have been desirable to reduce the coarse fraction in the FB batch wise granulate.

The Heinen granulates contain a much smaller coarse fraction compared to granulates from a batch process and are thus better suited with respect to the blending efficiency and prevention of subsequent segregation.

### Reference example 1

The same composition of the preblend of calcium carbonate and sorbitol as described under "materials end methods" was used in the following qualification trials with calcium carbonate, Merck 102064.
The qualification trials were run on an Aeromatic size 6 batch fluid bed with a batch size of 250 kg.

The most important process parameters controlling the agglomeration process in a batch fluid bed are the inlet air temperature, spray rate and inlet air humidity. The existing set-points based on the granulation of Scoralite 1A+1 B were 50°C and 720 g/ml for the inlet air granulation temperature and spray rate respectively. The inlet air humidity was ambient according to the weather conditions from the outside air. Variations in the inlet air humidity were compensated by adjusting the PVP concentration and thus keeping the moisture load constant during the trials.

It was quickly realized that the existing set of set-points for the inlet air temperature and spray rate did not produce a calcium granulate with satisfactory granulate characteristics. The resultant granulate had a much to high proportion of fine particles exceeding 50% below 125 µm. Increasing the spray rate and thus the moisture load in order to increase the mean particle size resulted in a too large proportion of large particles without decreasing the amount of fines significantly. This indicated an uneven distribution of the granulation liquid at these processing conditions.

Altogether 24 batches were run in order to define a new set of processing conditions where it was found to be necessary to reduce the inlet air granulation temperature from 50°C to 38°C. This results in a reduction of the product temperature during granulation from 24-26°C to 20-24°C. The relative humidity inside the product container during the granulation step increases when the product temperature decreases when keeping the moisture load constant. This results in a more sticky granulate and a danger of over wetting and lump formation in the product container. It also stresses the importance of being very careful with the monitoring of the absolute humidity in the inlet processing air and the necessity of frequent adjustments of the PVP concentration in the granulation liquid to compensate for these variations.

All in all the calcium carbonate, Merck 102064 is a difficult quality to run in a batch fluid bed. The processing difficulties must be related to the physical properties of the calcium carbonate particles where the shape and surfaces of the primary particles requires a different set of set-points for the critical processing parameters.

### Conclusion from the trials relating to the process according to the invention

Processing parameters: The most favourable set points for the critical process variables in Heinen continuous fluidised bed plant WT 4/13 were as follows:
- Inlet air volume: 1000 m³/h (at approx. 35 °C)
- Absolute moisture content of inlet air: 4 g/kg
- Concentration of PVP in granulation liquid: 15%
- Feed rate of powder mixture: 75 kg/h
- Retention time 1 hour
- Spray rate with three nozzles: 187. 5 g/min
- Inlet air temperature in all three inlet air compartments: 80°C

Control of mean particle size and distribution: The trials have shown that the mean particle size can be effectively controlled in a particle size window ranging from 100 to 300 µm and with a narrow size distribution documented by a low span values. This control over the mean particle size and the same time keeping a narrow size distribution facilitates a perfect matching of the calcium granulate to the mean particle size and distribution for a low amount of vitamin D₃ in the secondary granulate. The perfect matching of the two components during the blending step is documented by the good homogeneity of vitamin D₃ in the resultant chewable calcium tablets.

Robustness: The continuous fluid bed process have been show to be much more robust in several aspects connected to the granulation and drying of a calcium granulate as opposed to the batch process.

The continuous fluid bed process is more robust with respect to processing difficulties like clogging of powder on to product container screen, walls in the product container and expansion chamber and, clogging of exhaust air filters.
The continuous process is not sensitive to batch to batch variations due to frequent changes in the set-points for the critical processing parameters and is also less susceptible to changes in the inlet air moisture compared to a batch process. Finally it has been shown that the continuous process is much more robust to changes in the physical characteristics of calcium-containing compound.

## Claims

1. A process for producing a particulate material having a content of calcium carbonate of at least 40%, the process comprises granulating a fluidized composition comprising calcium carbonate optionally together with one or more pharmaceutically acceptable excipients under fluidizing conditions in a continuous fluid bed apparatus.

2. A process according to claim 1, wherein the granulation is performed by means of a granulation liquid that is applied to the fluidized composition comprising calcium carbonate.

3. A process according to claim 2, wherein the granulation liquid comprises a pharmaceutically acceptable binder.

4. A process according to claim 2, wherein the composition comprising calcium carbonate comprises a pharmaceutically acceptable binder.

5. A process according to any of claims 2-4, wherein the granulation liquid comprises a pharmaceutically acceptable solvent.

6. A process according to claim 5, wherein the solvent is water.

7. A process according to any of the preceding claims comprising the steps of
i) continuously feeding the composition to a zone of the continuous fluid bed apparatus with a feed rate (kg/h),
ii) continuously transferring the fluidized composition throughout one or more zones of the continuous fluid bed apparatus with a rate corresponding to that of the feed rate,
iii) continuously wetting the composition by spraying a granulation liquid to the fluidized composition with a spray load (kg solvent/h),
iv) continuously drying the wetted composition, and
v) continuously collecting the thus obtained particulate material with an output rate corresponding to that of the feed rate.

8. A process according to claim 7, wherein the steps are performed in two or more zones of the continuous fluid bed apparatus.

9. A process according to claim 7 or 8, wherein step i) and iv) are performed in different zones of the continuous fluid bed apparatus.

10. A process according to any of claims 7-9, wherein step iii) and iv) are performed in different zones of the continuous fluid bed apparatus.

11. A process according to any of the preceding claims, wherein the particulate material obtained has a SPAN value of at the most about 2.3 such as, e.g., at the most about 2.25, at the most about 2.1, at the most about 2 or at the most about 1.9.

12. A process according to any of the preceding claims, wherein the process is relatively robust with respect to set-point processing parameters towards changes in the mean particle size of the particular calcium carbonate employed.

13. A process according to any of the preceding claims, wherein the process is relatively robust with respect to set-point processing parameters towards changes in the bulk density of the particular calcium carbonate employed.

14. A process according to any of the preceding claims, wherein the particulate material obtained has a SPAN value of at the most about 2.3 such as, e.g., at the most about 2.25, at the most about 2.1 or at the most about 2 irrespective of the bed size of the continuous fluid bed apparatus employed, provided that the composition of the particular particulate material is the same and the ratio between the feed rate (kg/h) and the spray load (kg/h) is kept substantially constant.

15. A process according to any of the preceding claims, wherein up- or down-scaling between different continuous fluid bed equipment sizes is carried out by keeping one or more of the following set-point processing parameters constant
i) air velocity,
ii) inlet air temperature,
iii) inlet air humidity,
iv) bed height,
v) feed rate (kg/h)/spray load (kg solvent/h),
vi) atomizing pressure for the nozzle(s) employed,
vii) number of nozzles/product screen area.

16. A process according to claim 15, wherein two or more of the set-point processing parameters are kept constant during up- or down-scaling.

17. A process according to claim 16, wherein 3 or more such as, e.g., 4 or more, 5 or more, 6 or more or all set-point processing parameters are kept constant during up- or down-scaling.

18. A process according to claim 17, wherein all set-point processing parameters are kept constant during up- or down-scaling.

19. A process according to any of the preceding claims, wherein the particulate material obtained has a SPAN value of at the most about 2.3 such as, e.g., at the most about 2.25, at the most about 2.1, at the most about 2 or at the most about 1.9 irrespective of the particle size of the particular calcium carbonate employed provided that all other conditions including the set-points for processing parameters are substantially identical.

20. A process according to any of the preceding claims, wherein the particulate material obtained has a SPAN value of at the most about 2.3 such as, e.g., at the most about 2.25, at the most about 2.1, at the most about 2 or at the most about 1.9 irrespective of the bulk density of the particular calcium carbonate employed provided all other conditions including the set-points for processing parameters are substantially identical.

21. A process according to any of the preceding claims, wherein the granulated composition obtained under steady state conditions has a SPAN value that is smaller than that obtained when granulating the same composition with the same granulation liquid, but in a batch fluid bed apparatus.

22. A process according to claim 21, wherein the SPAN value obtained is about 10% or more smaller than that obtained using a batch fluid bed apparatus.

23. A process according to any of the preceding claims, wherein the bulk density of the calcium carbonate is less than about 1.5 g/ml.

24. A process according to any of the preceding claims, wherein the mean particle size of the calcium carbonate employed is less than about 60 µm.

25. A process according to any of the preceding claims, wherein the ratio between the feed rate (kg/h) of the composition comprising calcium carbonate and the spray load (kg solvent/h) of the granulation liquid is in a range of from about 4.5 to about 45 such as, e.g. from about 5 to about 30, from about 5 to about 20, from about 5 to about 15, from about 6 to about 14, from about 6 to about 12, from about 6 to about 10, from about 6.5 to about 8.5 or from about 7 to about 8.

26. A process according to any of the preceding claims, wherein the mean particle size of the particulate material obtained is controlled by the spray load and/or the moisture content of inlet air.

27. A process according to any of claims 1-25, wherein the mean particle size of the particulate material obtained is controlled by the spray load and the moisture content of inlet air is constant.

28. A process according to claim 26 or 27, wherein the mean particle size of the particulate material obtained increases by increasing spray load.

29. A process according to any of the preceding claims, wherein the mean particle size of the particulate material obtained is in a range of from about 100 to about 500 µm such as, e.g., from about 100 to about 400 µm, from about 100 to about 350 µm or from about 100 to about 300 µm.

30. A process according to any of claims 3-29, wherein the particulate material comprises
i) calcium carbonate,
ii) one or more binders
iii) optionally, one or more pharmaceutically acceptable excipients
iv) optionally, one or more sweetening agents.

31. A process according to claim 30, wherein the particulate material comprises
i) from about 40% to about 99.9% w/w of calcium carbonate,
ii) from about 0.1 % to about 30% w/w of one or more binders
iii) from about 0.1 to about 15% w/w of one or more pharmaceutically acceptable excipients, if present, and
iv) from about 5% to about 50% w/w of one or more sweetening agents, if present, provided that the total concentration does not exceed 100%.

32. Use of a particulate material as obtained by a process as defined in any of claims 1-31 for the preparation of a dosage form.

33. Use of a particulate material as obtained by a process as defined in any of claims 1-31 together with a vitamin D containing composition for the preparation of a dosage form.

## Patentansprüche

1. Verfahren zur Herstellung eines teilchenförmigen Materials, das einen Gehalt von Calciumcarbonat von mindestens 40 % aufweist, wobei das Verfahren das Granulieren einer verwirbelten Zusammensetzung, die Calciumcarbonat gegebenenfalls zusammen mit einem oder mehreren pharmazeutisch annehmbaren Arzneimittelhilfsstoffen umfasst, unter verwirbelten Bedingungen in einer kontinuierlichen Wirbelbettvorrichtung.

2. Verfahren gemäß Anspruch 1, wobei das Granulieren mittels einer Granulierungsflüssigkeit durchgeführt wird, die auf die verwirbelte Zusammensetzung, die Calciumcarbonat umfasst, aufgetragen wird.

3. Verfahren gemäß Anspruch 2, wobei die Granulierungsflüssigkeit ein pharmazeutisch annehmbares Bindemittel umfasst.

4. Verfahren gemäß Anspruch 2, wobei die Zusammensetzung, die Calciumcarbonat umfasst, ein pharmazeutisch annehmbares Bindemittel umfasst.

5. Verfahren gemäß einem der Ansprüche 2 bis 4, wobei die Granulierungsflüssigkeit ein pharmazeutisch annehmbares Lösungsmittel umfasst.

6. Verfahren gemäß Anspruch 5, wobei das Lösungsmittel Wasser ist.

7. Verfahren gemäß einem der vorstehenden Ansprüche, das die folgenden Schritte umfasst:
i) kontinuierliches Zuführen der Zusammensetzung in eine Zone der kontinuierlichen Wirbelbettvorrichtung mit einer Zuführungsgeschwindigkeit (kg/h);
ii) kontinuierliches Übertragen der verwirbelten Zusammensetzung durch eine oder mehrere Zonen der kontinuierlichen Wirbelbettvorrichtung mit einer Geschwindigkeit, die der Zuführungsgeschwindigkeit entspricht;
iii) kontinuierliches Benetzen der Zusammensetzung durch Aufsprühen einer Granulierungsflüssigkeit auf die verwirbelte Zusammensetzung mit einer Sprühlast (kg Lösungsmittel/h);
iv) kontinuierliches Trocknen der benetzten Zusammensetzung; und
v) kontinuierliches Auffangen des so erhaltenen teilchenförmigen Materials mit einer Austragsgeschwindigkeit, die der Zuführungsgeschwindigkeit entspricht.

8. Verfahren gemäß Anspruch 7, wobei die Schritte in zwei oder mehr Zonen der kontinuierlichen Wirbelbettvorrichtung durchgeführt werden.

9. Verfahren gemäß Anspruch 7 oder 8, wobei Schritt i) und iv) in verschiedenen Zonen der kontinuierlichen Wirbelbettvorrichtung durchgeführt werden.

10. Verfahren gemäß einem der Ansprüche 7 bis 9, wobei Schritt iii) und iv) in verschiedenen Zonen der kontinuierlichen Wirbelbettvorrichtung durchgeführt werden.

11. Verfahren gemäß einem der vorstehenden Ansprüche, wobei das erhaltene teilchenförmige Material einen SPAN-Wert von höchstens etwa 2,3 hat, wie zum Beispiel höchstens etwa 2,25, höchstens etwa 2,1, höchstens etwa 2 oder höchstens etwa 1,9.

12. Verfahren gemäß einem der vorstehenden Ansprüche, wobei das Verfahren bezüglich der Sollwert-Verarbeitungsparameter gegenüber Veränderungen in der mittleren Teilchengröße des eingesetzten teilchenförmigen Calciumcarbonats relativ robust ist.

13. Verfahren gemäß einem der vorstehenden Ansprüche, wobei das Verfahren bezüglich der Sollwert-Verarbeitungsparameter gegenüber Veränderungen in der Schüttdichte des eingesetzten teilchenförmigen Calciumcarbonats relativ robust ist.

14. Verfahren gemäß einem der vorstehenden Ansprüche, wobei das erhaltene teilchenförmige Material unabhängig von der Bettgröße der eingesetzten kontinuierlichen Wirbelbettvorrichtung einen SPAN-Wert von höchstens etwa 2,3 hat, wie zum Beispiel höchstens etwa 2,25, höchstens etwa 2,1 oder höchstens etwa 2, mit der Maßgabe, dass die Zusammensetzung des besonderen teilchenförmigen Materials dieselbe ist und das Verhältnis zwischen der Zuführungsgeschwindigkeit (kg/h) und der Sprühlast (kg/h) im Wesentlichen konstant gehalten wird.

15. Verfahren gemäß einem der vorstehenden Ansprüche, wobei eine Hoch- oder Herunterskalierung zwischen verschiedenen Größen der kontinuierlichen Wirbelbettvorrichtung durchgeführt wird, indem man einen oder mehrere de folgenden Sollwert-Verarbeitungsparameter konstant hält:
i) Luftgeschwindigkeit;
ii) Einlasslufttemperatur;
iii) Einlassluftfeuchtigkeit;
iv) Betthöhe;
v) Zuführungsgeschwindigkeit (kg/h)/Sprühlast (kg Lösungsmittel/h);
vi) Zerstäubungsdruck für die eingesetzten Düsen;
vii) Zahl der Düsen/Produktsiebfläche.

16. Verfahren gemäß Anspruch 15, wobei während der Hoch- oder Herunterskalierung zwei oder mehr Sollwert-Verarbeitungsparameter konstant gehalten werden.

17. Verfahren gemäß Anspruch 16, wobei während der Hoch- oder Herunterskalierung 3 oder mehr, wie zum Beispiel 4 oder mehr, 5 oder mehr, 6 oder mehr oder alle Sollwert-Verarbeitungsparameter konstant gehalten werden.

18. Verfahren gemäß Anspruch 17, wobei während der Hoch- oder Herunterskalierung alle Sollwert-Verarbeitungsparameter konstant gehalten werden.

19. Verfahren gemäß einem der vorstehenden Ansprüche, wobei das erhaltene teilchenförmige Material unabhängig von der Teilchengröße des eingesetzten teilchenförmigen Calciumcarbonats einen SPAN-Wert von höchstens etwa 2,3 hat, wie zum Beispiel höchstens etwa 2,25, höchstens etwa 2,1, höchstens etwa 2 oder höchstens etwa 1,9, mit der Maßgabe, dass alle anderen Bedingungen einschließlich der Sollwerte für Verarbeitungsparameter im Wesentlichen identisch sind.

20. Verfahren gemäß einem der vorstehenden Ansprüche, wobei das erhaltene teilchenförmige Material unabhängig von der Schüttdichte des eingesetzten teilchenförmigen Calciumcarbonats einen SPAN-Wert von höchstens etwa 2,3 hat, wie zum Beispiel höchstens etwa 2,25, höchstens etwa 2,1, höchstens etwa 2 oder höchstens etwa 1,9, mit der Maßgabe, dass alle anderen Bedingungen einschließlich der Sollwerte für Verarbeitungsparameter im Wesentlichen identisch sind.

21. Verfahren gemäß einem der vorstehenden Ansprüche, wobei die erhaltene granulierte Zusammensetzung unter stationären Bedingungen einen SPAN-Wert hat, der kleiner ist als der SPAN-Wert, den man erhält, wenn man dieselbe Zusammensetzung mit derselben Granulierungsflüssigkeit, aber in einer diskontinuierlichen Wirbelbettvorrichtung granuliert.

22. Verfahren gemäß Anspruch 21, wobei der erhaltene SPAN-Wert um etwa 10% oder mehr kleiner ist als der SPAN-Wert, den man unter Verwendung einer diskontinuierlichen Wirbelbettvorrichtung erhält.

23. Verfahren gemäß einem der vorstehenden Ansprüche, wobei die Schüttdichte des Calciumcarbonats kleiner als etwa 1,5 g/ml ist.

24. Verfahren gemäß einem der vorstehenden Ansprüche, wobei die mittlere Teilchengröße des eingesetzten Calciumcarbonats kleiner als etwa 60 µm ist.

25. Verfahren gemäß einem der vorstehenden Ansprüche, wobei das Verhältnis zwischen der Zuführungsgeschwindigkeit (kg/h) der Zusammensetzung, die Calciumcarbonat umfasst, und der Sprühlast (kg Lösungsmittel/h) der Granulierungsflüssigkeit in einem Bereich von etwa 4,5 bis etwa 45 liegt, wie zum Beispiel von etwa 5 bis etwa 30, von etwa 5 bis etwa 20, von etwa 5 bis etwa 15, von etwa 6 bis etwa 14, von etwa 6 bis etwa 12, von etwa 6 bis etwa 10, von etwa 6,5 bis etwa 8,5 oder von etwa 7 bis etwa 8.

26. Verfahren gemäß einem der vorstehenden Ansprüche, wobei die mittlere Teilchengröße des erhaltenen teilchenförmigen Materials durch die Sprühlast und/oder den Feuchtigkeitsgehalt der Einlassluft gesteuert wird.

27. Verfahren gemäß einem der Ansprüche 1 bis 25, wobei die mittlere Teilchengröße des erhaltenen teilchenförmigen Materials durch die Sprühlast gesteuert wird und der Feuchtigkeitsgehalt der Einlassluft konstant ist.

28. Verfahren gemäß Anspruch 26 oder 27, wobei die mittlere Teilchengröße des erhaltenen teilchenförmigen Materials bei zunehmender Sprühlast zunimmt.

29. Verfahren gemäß einem der vorstehenden Ansprüche, wobei die mittlere Teilchengröße des erhaltenen teilchenförmigen Materials in einem Bereich von etwa 100 bis etwa 500 µm liegt, wie zum Beispiel etwa 100 bis etwa 400 µm, etwa 100 bis etwa 350 µm oder etwa 100 bis etwa 300 µm.

30. Verfahren gemäß einem der Ansprüche 3 bis 29, wobei das teilchenförmige Material Folgendes umfasst:
i) Calciumcarbonat;
ii) ein oder mehrere Bindemittel;
iii) gegebenenfalls ein oder mehrere pharmazeutisch annehmbare Arznei mittelhilfsstoffe;
iv) gegebenenfalls ein oder mehrere Süßungsmittel.

31. Verfahren gemäß Anspruch 30, wobei das teilchenförmige Material Folgendes umfasst:
i) etwa 40 bis etwa 99,9 Gew.-% von Calciumcarbonat;
ii) etwa 0,1 bis etwa 30 Gew.-% eines oder mehrerer Bindemittel;
iii) etwa 0,1 bis etwa 15 Gew.-% eines oder mehrerer pharmazeutisch annehmbarer Arzneimittelhilfsstoffe, falls vorhanden; und
iv) etwa 5 bis etwa 50 Gew.-% eines oder mehrerer Süßungsmittel, falls vorhanden, mit der Maßgabe, dass die Gesamtkonzentration 100% nicht überschreitet.

32. Verwendung eines teilchenförmigen Materials, wie es nach einem Verfahren gemäß einem der Ansprüche 1 bis 31 erhalten wurde, zur Herstellung einer Darreichungsform.

33. Verwendung eines teilchenförmigen Materials, wie es nach einem Verfahren gemäß einem der Ansprüche 1 bis 31 erhalten wurde, zusammen mit einer Vitamin-D-haltigen Zusammensetzung zur Herstellung einer Darreichungsform.

## Revendications

1. Un procédé de production d'un matériau particulaire présentant une teneur du carbonate de calcium d'au moins 40%, ce procédé comprenant la granulation d'une composition fluidisée comprenant le carbonate de calcium, éventuellement en association avec un ou plusieurs excipients pharmaceutiquement acceptables, dans des conditions de fluidisation dans un appareil à lit fluidisé opérant en continu.

2. Un procédé selon la revendication 1, dans lequel la granulation est réalisée en présence d'un liquide de granulation qui est ajouté à la composition fluidisée comprenant le carbonate de calcium.

3. Un procédé selon la revendication 2, dans lequel le liquide de granulation comprend un liant pharmaceutiquement acceptable.

4. Un procédé selon la revendication 2, dans lequel la composition comprenant le carbonate de calcium comprend un liant pharmaceutiquement acceptable.

5. Un procédé selon une quelconque des revendications 2-4, dans lequel le liquide de granulation comprend un solvant pharmaceutiquement acceptable.

6. Un procédé selon la revendication 5, dans lequel le solvant est de l'eau.

7. Un procédé selon une quelconque des revendications précédentes comprenant les étapes suivantes:
i) l'alimentation en continu de la composition dans une zone de l'appareil à lit fluidisé opérant en continu selon une vitesse d'alimentation (kg/h),
ii) le transfert en continu de la composition fluidisée au travers de une ou plusieurs zones de l'appareil à lit fluidisé opérant en continu selon une vitesse correspond à celle de la vitesse d'alimentation,
iii) l'humidification en continu de la composition par pulvérisation d'un liquide de granulation dans la composition fluidisée sous une charge de pulvérisation, (kg solvant/h),
iv) le séchage en continu de la composition humide, et
v) la récupération en continu de la matière particulaire ainsi obtenue selon un débit de production correspondant à celui de la vitesse d'alimentation

8. Un procédé selon la revendication 7, dans lequel lesdites étapes sont effectuées dans deux ou plusieurs zones de l'appareil à lit fluidisé opérant en continu.

9. Un procédé selon la revendication 7 ou 8, dans lequel les étapes i) et iv) sont effectuées dans différentes zones de l'appareil à lit fluidisé opérant en continu.

10. Un procédé selon une quelconque des revendications 7-9, dans lequel les étapes iii) et iv) sont effectuées dans différentes zones de l'appareil à lit fluidisé opérant en continu.

11. Un procédé selon une quelconque des revendications précédentes, dans lequel le matériau particulaire obtenu présente une valeur SPAN au maximum d'environ 2,3 à savoir, par exemple, au maximum d'environ 2,25, au maximum d'environ 2,1, au maximum d'environ 2 ou au maximum d'environ 1,9.

12. Un procédé selon une quelconque des revendications précédentes, dans lequel ce procédé est relativement robuste à l'égard des paramètres de traitement au point de réglage par rapport aux variations de la dimension particulaire moyenne du carbonate de calcium particulaire employé.

13. Un procédé selon une quelconque des revendications précédentes, dans lequel ce procédé est relativement robuste à l'égard des paramètres de traitement au point de réglage par rapport aux variations à la densité apparente du carbonate de calcium particulaire employé.

14. Un procédé selon une quelconque des revendications précédentes, dans lequel le matériau particulaire obtenu présente une valeur SPAN au plus d'environ 2,3 telle que, par exemple, au plus d'environ 2,25, au plus d'environ 2,1 ou au plus d'environ 2 indépendamment de la dimension du lit de l'appareil à lit fluidisé opérant en continu employé, à la condition que la composition du matériau particulaire particulier soit la même et que le rapport vitesse d'alimentation (kg/h)/charge de pulvérisation (kg/h) soit maintenu pratiquement constant.

15. Un procédé selon une quelconque des revendications précédentes, dans lequel la conversion ascendante et la conversion descendante entre différentes dimensions d'appareillage à lit fluidisé opérant en continu est mis en oeuvre par maintien de un ou plusieurs des paramètres de traitement au point de réglage constant suivants:
i) vitesse de l'air,
ii) température d'entrée de l'air,
iii) humidité de l'air d'entrée,
iv) hauteur du lit,
v) vitesse d'alimentation (kg/h)/charge de pulvérisation (kg solvant/h),
vi) pression d'atomisation pour la ou les tuyères employées,
vii) nombre de tuyères/aire de l'écran de produit.

16. Un procédé selon la revendication 15, dans lequel deux ou plus des paramètres de traitement au point de réglage sont maintenus constants durant les conversions ascendante et descendante.

17. Un procédé selon la revendication 16, dans lequel au moins 3, à savoir, par exemple, 4 ou davantage, 5 ou davantage, 6 ou davantage ou tous les paramètres de traitement au point de réglage sont maintenus constants durant les conversions ascendante et descendante.

18. Un procédé selon la revendication 17, dans lequel tous les paramètres de traitement au point de réglage sont , les conversions ascendante et descendante.

19. Un procédé selon une quelconque des revendications précédentes, dans lequel le matériau particulaire obtenu présente une valeur SPAN au plus d'environ 2,3 à savoir, p. ex., au plus d'environ: 2,25, au plus d'environ 2,1, au plus d'environ 2 ou au plus d'environ 1,9, indépendamment de la dimension particulaire du carbonate de calcium particulier employé, à la condition que toutes les autres conditions incluant le point de réglage des paramètres de traitement soient pratiquement identiques.

20. Un procédé selon une quelconque des revendications précédentes, dans lequel le matériau particulaire obtenu présente une valeur SPAN au plus d'environ 2,3, à savoir, p. ex., au plus d'environ 2,25, au plus d'environ 2,1, au plus d'environ 2 ou au plus d'environ 1,9 indépendamment de la densité apparente du carbonate de calcium particulier employé, à la condition que toutes les autres conditions incluant le point de réglage des paramètres de traitement soient pratiquement identiques

21. Un procédé selon une quelconque des revendications précédentes, dans lequel la composition granulée obtenue dans des conditions de régime permanent présente une valeur SPAN qui est inférieure à celle que l'on obtient quand on granule la même composition avec le même liquide de granulation, mais dans un appareil à lit fluidisé opérant en discontinu.

22. Un procédé selon la revendication 21, dans lequel la valeur de SPAN obtenue est d'environ d'au moins 10% inférieure à celle obtenue en faisant emploi d'un appareil à lit fluidisé opérant en discontinu.

23. Un procédé selon une quelconque des revendications précédentes, dans lequel la densité apparente du carbonate de calcium est inférieure à environ 1,5 g/ml.

24. Un procédé selon une quelconque des revendications précédentes, dans lequel la dimension particulaire moyenne du carbonate de calcium employé est inférieure à environ 60 µm.

25. Un procédé selon une quelconque des revendications précédentes, dans lequel le rapport entre la vitesse d'alimentation (kg/h) de la composition comprenant le carbonate de calcium et la charge de pulvérisation (kg solvant/h) du liquide de granulation est compris entre environ 4,5 et environ 45 à savoir, par exemple entre environ 5 et environ 30, entre environ 5 et environ 20, entre environ 5 et environ 15, entre environ 6 et environ 14, entre environ 6 et environ 12, entre environ 6 et environ 10, entre environ 6.5 et environ 8.5 ou entre environ 7 et environ 8.

26. Un procédé selon une quelconque des revendications précédentes, dans lequel la dimension particulaire moyenne du matériau particulaire obtenu est asservi à la charge de pulvérisation et/ou la teneur en humidité de l'air d'entrée.

27. Un procédé selon une quelconque des revendications 1-25, dans lequel la dimension particulaire moyenne du matériau particulaire obtenu est asservi à la charge de pulvérisation et dans lequel la teneur en humidité de l'air d'entrée est constant.

28. Un procédé selon la revendication 26 ou 27, dans lequel la dimension particulaire moyenne du matériau particulaire obtenu croit avec l'augmentation de la charge de pulvérisation.

29. Un procédé selon une quelconque des revendications précédentes, dans lequel la dimension particulaire moyenne du matériau particulaire obtenu est compris entre environ 100 et environ 500 µm, à savoir, par exemple, entre environ 100 et environ 400 µm, entre environ 100 et environ 350 µm ou entre environ 100 et environ 300 µm.

30. Un procédé selon une quelconque des revendications 3-29, dans lequel le matériau particulaire comprend
i) le carbonate de calcium,
ii) un ou plusieurs liants
iii) éventuellement un ou plusieurs excipients pharmaceutiquement acceptables
iv) éventuellement, un ou plusieurs agents édulcorants.

31. Un procédé selon la revendication 30, dans lequel le matériau particulaire comprend
i) d'environ 40% à environ 99,9% pds/pds du carbonate de calcium,
ii) d'environ 0,1% à environ 30% pds/pds de un ou plusieurs liants
iii) d'environ 0.1 à environ 15% pds/pds de un ou plusieurs excipients pharmaceutiquement acceptables, s'il en est présent, et
iv) d'environ 5% à environ 50% pds/pds de un ou plusieurs agents édulcorants, s'il en est présent, à la condition que la concentration totale ne dépasse pas 100%.

32. Emploi d'un matériau particulaire tel qu'obtenu par un procédé tel que défini dans une quelconque des revendications 1 à 31 pour la préparation d'une forme pharmaceutique.

33. Emploi d'un matériau particulaire tel qu'obtenue par un procédé tel que défini dans une quelconque des revendications 1 à 31, associé à une composition contenant une vitamine D pour la préparation d'une forme pharmaceutique.
